# EUROPEAN PATENT SPECIFICATION

(11) **EP 3 237 908 B1**
(45) Date of publication and mention of the grant of the patent: **22.04.2026**
(21) Application number: 15817838.4
(22) Date of filing: 21.12.2015
(51) Int. Cl.: C12N 15/10, G01N 33/566

(54) **DIRECTED EVOLUTION OF MEMBRANE PROTEINS IN EUKARYOTIC CELLS WITH A CELL WALL**
GERICHTETE EVOLUTION VON MEMBRANPROTEINEN IN EUKARYOTISCHEN ZELLEN MIT ZELLWAND
L'ÉVOLUTION DIRIGÉE DES PROTÉINES MEMBRANAIRES DANS DES CELLULES EUCARYOTES AYANT UNE PAROI CELLULAIRE

(30) Priority: 22.12.2014 EP 14199729
(43) Date of publication of application: 01.11.2017
(73) Proprietor: Universität Zürich, 8006 Zürich (CH)
(72) Inventor: SCHÜTZ, Marco, CH-8037 Zürich (CH); PLÜCKTHUN, Andreas, CH-8006 Zürich (CH)
(74) Representative: Gill Jennings & Every LLP
(86) International application number: PCT/EP2015/080858
(87) International publication number: WO 2016/102508

(56) References cited:
- EP-A1- 2 612 916
- WO-A2-2008/100816
- US-A1- 2003 232 395
- US-B1- 6 696 251
- ADDISON D. AULT ET AL: "Creation of GPCR-based chemical sensors by directed evolution in yeast", PROTEIN ENGINEERING, DESIGN AND SELECTION, vol. 19, no. 1, 1 November 2005 (2005-11-01), GB, pages 1 - 8, XP055587585, ISSN: 1741-0126, DOI: 10.1093/protein/gzi069
- "METHODS IN ENZYMOLOGY", vol. 520, 1 January 2013, ACADEMIC PRESS, US, ISSN: 0076-6879, article KAROLA M. SCHLINKMANN ET AL: "Directed Evolution of G-Protein-Coupled Receptors for High Functional Expression and Detergent Stability", pages: 67 - 97, XP055248661, DOI: 10.1016/B978-0-12-391861-1.00004-6
- AZADEH POURMIR ET AL: "DIRECTED EVOLUTION: SELECTION OF THE HOST ORGANISM", COMPUTATIONAL AND STRUCTURAL BIOTECHNOLOGY JOURNAL, vol. 2, no. 3, 1 September 2012 (2012-09-01), Sweden, pages 1 - 7, XP055248660, ISSN: 2001-0370, DOI: 10.5936/csbj.201209012
- DANIEL J. SCOTT ET AL: "Improving the apo-state detergent stability of NTS1 with CHESS for pharmacological and structural studies", BIOCHIMICA ET BIOPHYSICA ACTA (BBA) - BIOMEMBRANES, vol. 1838, no. 11, 9 February 2014 (2014-02-09), AMSTERDAM, NL, pages 2817 - 2824, XP055248657, ISSN: 0005-2736, DOI: 10.1016/j.bbamem.2014.07.015
- BEUKERS M W ET AL: "Techniques: How to boost GPCR mutagenesis studies using yeast", TRENDS IN PHARMACOLOGICAL SCIENCES, ELSEVIER, HAYWARTH, GB, vol. 26, no. 10, 1 October 2005 (2005-10-01), pages 533 - 539, XP027666801, ISSN: 0165-6147, [retrieved on 20051001]
- IGOR DODEVSKI ET AL: "Evolution of Three Human GPCRs for Higher Expression and Stability", JOURNAL OF MOLECULAR BIOLOGY, ACADEMIC PRESS, UNITED KINGDOM, vol. 408, no. 4, 22 February 2011 (2011-02-22), pages 599 - 615, XP028209045, ISSN: 0022-2836, [retrieved on 20110303], DOI: 10.1016/J.JMB.2011.02.051

## Description

Structural and detailed biochemical investigation of eukaryotic transmembrane receptors is still hampered despite technical advances in expression, purification and crystallization strategies. Important difficulties are the lacking ability to produce the desired receptors in sufficient amounts and their inherent instability.

A method to increase functional expression levels of GPCRs in *Escherichia coli* by directed evolution is known in the art (Sarkar et al. (2008), PNAS 105:14808-14813; Dodevski & Plückthun (2011), J Mol Biol 408:599-615). Several high-expression variants of GPCRs have been isolated with this method. However, since *E. coli* is not able to perform post-translational modifications, lacks the secretory quality control and translocation machinery for eukaryotic membrane proteins, and differs in membrane composition, evolution of eukaryotic transmembrane receptors in *E. coli* is limited to receptors which inherently express in a prokaryotic system above the threshold required for successful evolution, thus not having strict requirements for eukaryotic processing or membrane composition.

WO 2006/100816 describes a method for displaying antibodies on the extracellular surface of the plasma membrane of eukaryotic yeast cells. The expressed antibodies comprise transmembrane receptor proteins including a GPCR transmembrane domain to target them to the cell surface.

Therefore the establishment of a fast, efficient and robust method to increase functional expression of transmembrane receptors in a eukaryotic host is required. In principle the ideal eukaryotic host would be cells that are used for the production of the evolved high-expression variants. However, these mammalian or insect cells are not easily transformed with libraries, which is essential for any type of directed evolution approach. The usage of "lower" eukaryotes such as the yeast *Saccharomyces cerevisiae* is common for other procedures involving libraries. However, yeast cells comprise a cell wall, which limits functionality assays by restricting access of administered ligands to expressed receptors located in the plasma membrane. For soluble proteins, a common solution to this problem is the use of methods such as yeast display, which expresses the protein of interest as fusion protein that is presented outside of the cell wall. However, this approach is not suitable for selection of functional high-expression variants of insoluble transmembrane receptors, which must be located in the plasma membrane, and thus below the cell wall, and cannot be expressed as fusion proteins attached to the cell wall.

The problem underlying the present invention is to provide novel efficient methods to facilitate and accelerate research and drug design in transmembrane receptors, particularly by identifying transmembrane protein variants resulting in optimized functional expression in eukaryotic cells comprising a cell wall. This problem is solved by the subject-matter of the independent claims.

### Terms and definitions

In the context of the present specification, the term *alkaline pH* is used in its meaning known in the art of chemistry; it refers to a measure of the basicity of an aqueous solution. A pH greater than 7 indicates alkaline or basic solutions and a pH less than 7 indicates an acidic solution.

In the context of the present specification, the term *reducing agent* is used in its meaning known in the art of chemistry and biochemistry; it refers to an element or compound that donates an electron to another chemical substance in a redox reaction. In this process the reducing agent is oxidized.

In the context of the present specification, the terms *fluorescent cell sorting* or *fluorescence-activated cell sorting (FACS)* are used in their meaning known in the art of cell biology; they refer to a method for cell sorting, wherein cells are suspended in a stream of fluid and pass a detection device. Every passing cell can be directed into different compartments according to the intensity of a specific fluorescent signal of the passing cell.

In the context of the present specification, the term *random mutagenesis* is used in its meaning known in the art of cell biology and molecular biology; it refers to a method wherein DNA mutations are randomly introduced to produce mutant genes and proteins. A multitude of these mutant genes can then be compiled into a library. Non-limiting examples for random mutagenesis methods are error-prone PCR, UV radiation and chemical mutagens.

In the context of the present specification, the term *library* is used in its meaning known in the art of cell biology and molecular biology; it refers to a collection of nucleic acid fragments. One particular type of library is a randomized mutant library, generated by random mutagenesis. Another example would be a designed (or synthetic) library, which comprises specifically engineered DNA fragments.

In the context of the present specification, the term *expression level* is used in its meaning known in the art of cell biology and molecular biology; it refers to the level of transcription and/or translation of a DNA fragment and the derived mRNA, respectively. In certain embodiments an expression level is deemed to be *high* if the expression of a mutant gene is higher than a control gene or wild-type gene. In certain embodiments the expression of the mutant gene is at least two-fold higher than that of the control or wild-type gene to be deemed *high.*

According to a first aspect of the invention a method is provided for increasing expression of functional G protein-coupled receptors (GPCRs) in eukaryotic cells, wherein said functional GPCRs are capable of ligand binding.

The method comprises the steps of:
a) providing a plurality of yeast cells, wherein each of said yeast cells comprises a nucleic acid sequence member of a randomized mutant library, and said nucleic acid sequence member is expressed as a GPCR in the plasma membrane in said plurality of yeast cells
b) permeabilizing the cell wall of said plurality of yeast cells in a permeabilization step, wherein the permeabilization step comprises exposing the plurality of yeast cells to a chemical treatment, yielding a plurality of viable permeabilized cells, wherein the chemical treatment is a buffer of alkaline pH comprising lithium ions, a reducing agent and/or a chelating agent
c) contacting said plurality of viable permeabilized cells in a labelling step with a ligand capable of binding to said GPCR, wherein said ligand comprises a detectable label, yielding a plurality of viable labelled cells,
d) washing said plurality of viable labelled cells in a washing step,
e) selecting a subset of said plurality of viable labelled cells as a function of detectable label present in said plurality of viable labelled cells in a selection step, yielding a selection of viable cells, wherein said detectable label is a fluorescent dye and said selection step is accomplished by fluorescent cell sorting,
f) after said selection step e) said selection of viable cells is expanded in an expansion step, yielding an expanded selection of viable cells and said expanded selection of viable cells is subjected to said steps b) to e) in this sequential order,
g) repeating step f) at least 1, 2, 3, 4, 5, 6 or 7 times, and
h) isolating an expressed nucleic acid sequence from the expanded selection of viable cells of step f) in an isolation step,
wherein the expanded selection of viable cells of step g) has increased expression of functional GPCRs as compared to the plurality of eukaryotic yeast (claim 9) cells of step a).

In other words, the method according to the first aspect of the invention allows the expression of a library of GPCRs in eukaryotic cells comprising a cell wall, i. e. yeast cells, and allows for the selection of functional expression of these receptors. This is made possible by the permeabilization of the cell wall in a way that still maintains viable and structurally stable cells, which is different from other methods of permeabilization of the cell wall such as the preparation of spheroplasts. The permeabilization procedure of this invention allows even the binding of large ligands to the GPCRs. Selection of the cells by the amount of bound ligand enables selection of the cells according to the amount of functional receptor on the plasma membrane and not by total receptor amount, which would also include non-functional receptors (e.g. receptor present in intracellular membranes).

The selection step comprises a multitude of selection procedures. After a first selection the selection of cells is used again for the selection of a subset of these cells as a function of detectable label present in these cells. Selection of cells is repeated at least 1, 2, 3, 4 or 5 times.

The library of expressed nucleic acid sequences is obtained by amplification of a nucleic acid sequence encoding the GPCR by a process introducing mutations into the amplified sequence.

In certain embodiments the method additionally comprises the following steps:
i. the expressed nucleic acid obtained in isolation step h) is introduced and expressed in a plurality of yeast cells,
ii. said plurality of yeast cells is subjected to said steps a) to h) according to the first aspect of the invention, and
iii. steps i. and ii. are performed at least 1, 2, 3, 4, 5, 6 or 7 times.

In certain embodiments the expressed nucleic acid sequences obtained by the method of the invention are characterized by high thermodynamic stability of the encoded GPCR.

The selection of viable cells can comprise the top 0.1% to 5% of the most fluorescent cells.

In certain embodiments the method additionally comprises the following steps:
i. The expressed nucleic acid obtained in the isolation step h) is amplified by a process introducing mutations into the amplified sequence, yielding a second library of nucleic acid sequences.
ii. This second library of nucleic acid sequences is transferred to the plurality of yeast cells,
iii. The plurality of yeast cells, now comprising a nucleic acid sequence member of the second library, is submitted to the method according to the first aspect of this invention.

According to the invention eukaryotic cells comprising a cell wall are yeast cells.

An enzymatic treatment in a permeabilization step may comprise exposing a plurality of eukaryotic cells comprising a cell wall to enzymes or enzyme mixtures that permeabilize the cell wall. Non-limiting examples of such enzymes are glucanases, proteases, mannases and/or sulfatases. Non-limiting examples of such enzyme mixtures are Zymolyase, Lyticase, and/or Glusulase.

In certain embodiments non-limiting examples of buffering agents contained in the buffer used in the permeabilization step are:
- Bicine (2-(Bis(2-hydroxyethyl)amino)acetic acid) or
- HEPES (2-[4-(2-Hydroxyethyl)piperazin-1-yl]ethanesulfonic acid) or
- MOPS (3-morpholinopropane-1-sulfonic acid) or
- PIPES (1,4-Piperazinediethanesulfonic acid) or
- TAPS (3-[[1,3-Dihydroxy-2-(hydroxymethyl)propan-2-yl]amino]propane-1-sulfonic acid) or
- TAPSO (3-[[1,3-Dihydroxy-2-(hydroxymethyl)propan-2-yl]amino]-2-hydroxypropane-1-sulfonic acid) or
- TES (2-[[1,3-Dihydroxy-2-(hydroxymethyl)propan-2-yl]amino]ethanesulfonic acid) or
- Tricine (N-(2-Hydroxy-1,1-bis(hydroxymethyl)ethyl)glycine) or
- Tris (2-Amino-2-hydroxymethyl-propane-1,3-diol).

In certain embodiments the chelating agent used in the buffer of the permeabilization step is ethylenediaminetetraacetic acid (EDTA).

According to the invention the detectable label is a fluorescent dye and the selection step is accomplished by fluorescent cell sorting.

In certain embodiments non-limiting examples of reducing agent are:
- thiol-containing compounds, such as, dithiothreitol (DTT), dithioerythritol (DTE), mercaptoethanol or reduced glutathione or
- phosphine-containing compounds, such as tris-carboxyethyl-phosphine (TCEP).

In certain embodiments the permeabilization step comprises the following steps:
a) incubating the yeast cells in TELi buffer comprising 50 mM Tris-HCl pH 9, 1 mM EDTA and 100 mM lithium acetate,
b) incubating the yeast cells in TELi buffer additionally comprising 50 mM DTT for 30 min at 20°C,
c) washing the yeast cells at least once in TELi buffer at 4°C.

In certain embodiments TELi buffer comprises 50 mM Tris-HCl pH 9, 1 mM EDTA and 100 mM lithium acetate.

According to the invention the buffer used in the permeabilization step comprises:
i. lithium ions,
ii. alkaline pH,
iii. reducing agent, and/or
iv. chelating agent.

In certain embodiments the labeling step comprises exposing the yeast cells to TELi buffer comprising the ligand at 4°C.

In certain embodiments the nucleic acid sequences for the library of expressed nucleic acid sequences are generated by random mutagenesis preferably by error-prone PCR.

In certain embodiments the library of expressed nucleic acid sequences comprises homologous sequences of at least 60% sequence identity with each other.

The eukaryotic cell according to the invention is a yeast cell, particularly *Saccharomyces cerevisiae, Pichia pastoris, Kluyveromyces lactis, Candida boidinii,* or *Hansenula polymorpha.*

The eukaryotic cells comprising a cell wall are a mutant or genetically engineered yeast strain not comprising a native cell wall.

In certain embodiments the yeast cell is *Saccharomyces cerevisiae,* particularly the *S*. *cerevisiae* strain BY4741.

In certain embodiments the ligand specifically capable of binding to the target membrane protein is an agonist, antagonist or allosteric modulator.

In certain embodiments the ligand specifically capable of binding to the target membrane protein is an oligopeptide comprised of at least 3, 4, 5, 6, 8, 10, 14, 18 or 25 amino acids.

In other embodiments, the ligand specifically capable of binding to the receptor is an antibody, antibody fragment or another binding protein, e.g., a scaffold protein from the non-limiting list of examples of DARPins, affibodies, anticalins, nanobodies, affilins, fibronectin-derived scaffolds and other scaffolds.

The disclosure provides a method for selecting a sequence from a library of expressed nucleic acid sequences, wherein the sequence is selected according to its expression level. The method comprises the following steps.
a) A plurality of eukaryotic cells comprising a cell wall, particularly a plurality of yeast cells, is provided, wherein each of the eukaryotic cells comprises a nucleic acid sequence member of the library. The nucleic acid sequence member is a transgene to the cell, expressed under the control of a promoter sequence operable in the cell, as a target membrane protein in the plurality of eukaryotic cells comprising a cell wall.
b) The plurality of eukaryotic cells comprising a cell wall is contacted in a labelling step with a ligand specifically capable of binding to the target membrane protein. The ligand comprises a detectable label, yielding a plurality of labelled cells.
c) The plurality of labelled cells is washed in a washing step, thereby removing most of or any ligand and detectable label not having bound specifically to the target membrane protein.
d) The presence of said detectable label for each of the plurality of labelled cells is detected and a subset of the plurality of labelled cells is selected as a function of detectable label present in the plurality of labelled cells in a selection step. In other words, the cells that show any label, or a label quantity above a certain threshold is selected, yielding a selection of viable cells.
e) The expressed nucleic acid sequence from the selection of cells is isolated in an isolation step.

In other words, the method allows the expression of a library of transmembrane receptors in eukaryotic cells comprising a cell wall and allows for the selection of functional expression of these receptors. Selection of the cells by the amount of bound ligand enables selection of the cells according to the amount of functional receptor on the plasma membrane and not by total receptor amount, which would also include non-functional receptors (e.g. receptor present in intracellular membranes). This may be advantageous for the use of small ligands. According to a second aspect of the invention a method for the selection of an adapted yeast cell with the ability for high expression levels of functional GPCRs is provided. The method comprises the following steps:
a. providing a plurality of yeast cells, wherein each of said yeast cells comprises a nucleic acid sequence member of a randomized mutant library of expressed nucleic acid sequences, and said nucleic acid sequence member is expressed as a GPCR in the plasma membrane in said plurality of yeast cells,
b. permeabilizing the cell wall of said plurality of yeast cells in a permeabilization step, wherein the permeabilization step comprises exposing the plurality of yeast cells to a chemical treatment, yielding a plurality of viable permeabilized cells, wherein the chemical treatment is a buffer of alkaline pH comprising lithium ions, a reducing agent and/or a chelating agent,
c. contacting said plurality of viable permeabilized cells in a labelling step with a ligand capable of binding to said GPCR, wherein said ligand comprises a detectable label, yielding a plurality of viable labelled cells,
d. washing said plurality of viable labelled cells in a washing step,
e. selecting a subset of said plurality of viable labelled cells as a function of detectable label present in said plurality of viable labelled cells in a selection step, yielding a selection of viable cells,
   wherein said detectable label is a fluorescent dye and said selection step is accomplished by fluorescent cell sorting,
f. expanding said selection of viable cells in an expansion step, yielding an expanded selection of viable cells,
g. submitting said expanded selection of viable cells to steps b. to f. at least 1, 2, 3, 4, 5, 6 or 7 times,
h. submitting said expanded selection of viable cells to steps b. to e., and
i. selecting a subset of said expanded selection of viable cells as a function of detectable label present in said plurality of viable labelled cells, yielding said adapted yeast cell with the ability for high expression levels of functional GPCR from said expanded selection of cells.

### Short description of the figures

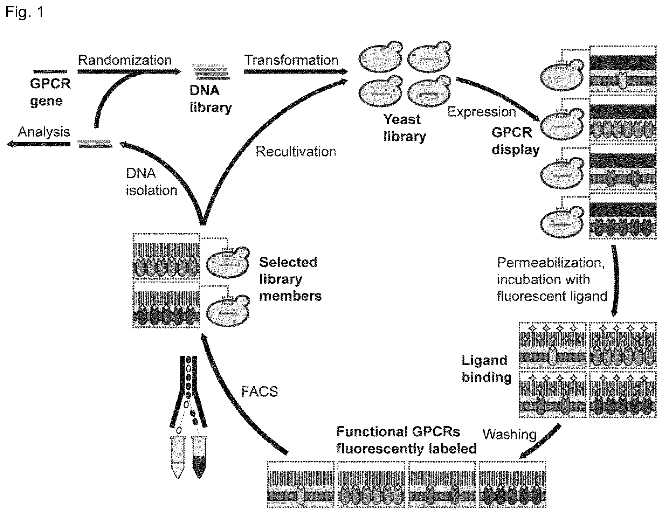

Fig. 1 shows the workflow for directed evolution of GPCRs in yeast. As a first step, the wild-type GPCR gene is randomized by error-prone PCR in order to create a DNA library. The DNA library is then combined with linearized yeast expression vector and the mixture is used for transformation of yeast. Insert DNA and vector backbone are assembled *in vivo* by homologous recombination. The obtained yeast library is cultivated and expression is induced. After expression, cells are permeabilized and incubated with fluorescent ligand, which binds exclusively to functional GPCRs in the plasma membrane. Subsequently, unbound ligand is removed by washing and cells are subjected to selection by FACS. Selection of cells exhibiting high fluorescence, correspondingly expressing GPCRs at high functional level, allows isolation of desired high-expression GPCR variants. During FACS, yeast cells are directly sorted into growth medium for subsequent propagation. Selection by FACS is repetitively performed in order to obtain a strong enrichment of cells harboring the best expressing GPCRs. Whenever desired, plasmid DNA can be isolated from selected cells for analysis of individual variants or additional diversity can be introduced by random mutagenesis for another round of evolution.

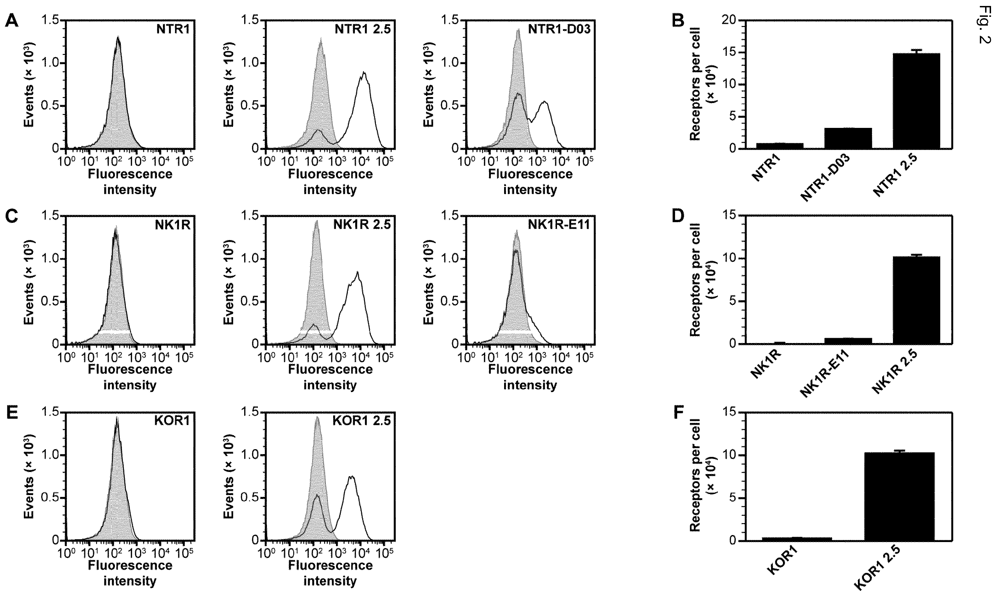

Fig. 2 shows *(A)*, *(C)*, *(E)* histogram plots of ligand-binding FC data of NTR1, NK1R, and KOR1 variants. Functional expression in yeast of wild-type GPCRs (left panels), library pools obtained after the second round of evolution (middle panel) and variants evolved in the *E*. *coli*-based system (right panels). Total signal was obtained by binding of fluorescent ligand to receptors in yeast cells expressing the corresponding GPCR variants (black curves). Nonspecific binding was measured in the presence of excess unlabeled ligand (gray, tinted). For the wild-type GPCRs no specific signal is detected due to the low functional expression levels at the surface. The selected library pools show high specific signals (functional receptors at the surface per cell) in expressing cells with a small fraction of cells not expressing any functional receptor at the surface (note double peak of total signal). Variants previously evolved in the *E*. *coli*-based system (NTR1-D03, NK1R-E11) show a specific signal, however not reaching the levels obtained for the yeast library pools. Furthermore, for a significantly higher fraction of cells no functional GPCR expression is detected at the surface. For instance, only about 50% of all cells measured show a specific signal for NTR1-D03 expression. *(B)*, *(D)*, *(F)* Measurement of average total functional receptors per cell by RLBA of NTR1, NK1R, and KOR1 variants. Expression levels were quantified with [³H]-radioligands. Non-specific signal was measured in the presence of [³H]-radioligands with excess of unlabeled ligand and was subtracted from the total signal. Wild-type NTR1 and KOR1 show low expression levels, while for NK1R no functional expression at all is detected. NTR1-D03 shows moderate receptor-per-cell levels, whereas for NK1RE11 expression levels are low. The selected library pools show functional production levels of 100,000 - 150,000 receptors per cell, an increase of 25 - 50-fold and 5 - 20-fold compared to the wild-type GPCRs and the variants evolved in *E. coli,* respectively. Error bars indicate standard deviations from triplicates of a representative expression experiment.

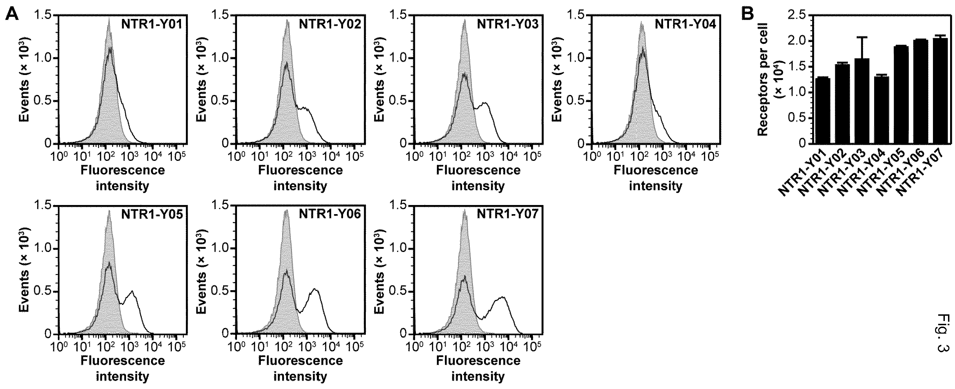

Fig. 3 shows functional expression of selected NTR1 variants (NTR1-Y01 - NTR1-Y07) in non-adapted yeast strains. *(A)* Histogram plots of ligand-binding FC data with total signal (black curves) and nonspecific signal (gray, tinted) are shown. Compared to wild-type NTR1 all selected variants show an increase in functional surface expression (cf. Fig. 2 *A*)*.* In comparison with the library pool NTR1 2.5, the individual variants depict an increase of the subpopulation not expressing functional receptor at the surface as well as lower specific signals (receptors per cell) in cells with surface expression of active receptor (cf. Fig. 2 *A*)*. (B)* Measurement of average total functional receptors per cell by RLBA. All variants show an increase in functional expression compared to wild-type NTR1, but do not reach the expression levels observed for the NTR1 2.5 pool (cf. Fig. 2 *B*). Non-specific signal was subtracted from total signal. Error bars indicate standard deviations from triplicates of a representative expression experiment.

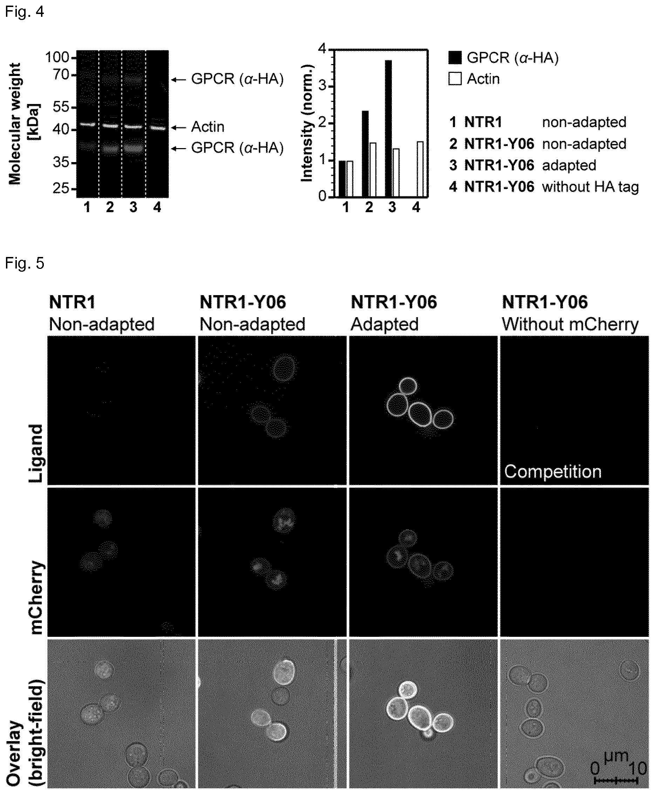

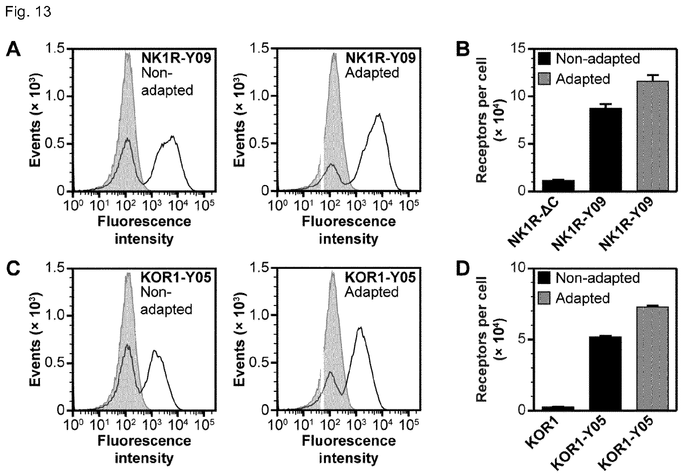

Fig. 4 shows quantitative Western blot analysis of HA-tagged NTR1 variants expressed in non-adapted and adapted yeast strains. Whole cell lysate protein extraction was performed with equal numbers of cells for each sample after expression. As a loading control, actin was used. GPCRs were detected via their HA tag, with main bands corresponding to monomeric GPCRs running slightly below the expected molecular size (44 kDa) and bands of higher molecular weight, most likely representing GPCR dimers not disintegrated under the conditions used. For quantification (bar chart) intensities of all defined bands were accounted for and signals were normalized to the GPCR and actin intensities obtained for wild-type NTR1. In the non-adapted strains, the total GPCR produced increases from wild-type NTR1 (lane 1) to the evolved variant (lane 2) by a factor of two. While total receptor levels of NTR1-Y06 also slightly increase in the adapted strain (lane 3) compared to the non-adapted strain (lane 2), the relative increase of total receptor produced is much lower than the increase in functional receptor (cf. Fig 13 *B*). For a negative control (lane 4), cells expressing NTR1-Y06 without HA tag were used.

Fig. 5 shows confocal fluorescence microscopy studies of NTR1 variants with C-terminal fusion to mCh expressed in non-adapted and adapted yeast strains. Fluorescence intensities obtained by binding of fluorescent ligand (top row) or from mCh (middle row) as well as bright-field microscopy overlays (bottom row) are shown. For expression of wild-type NTR1 (first column), no cells with a ligand-binding signal at the cell surface are detected, while some cells show distinct mCh signals, mainly located in the cell interior, and thus reflecting intracellularly retained receptors. Additionally, many cells lacking any fluorescent signal are detected, representing a non-expressing subpopulation. In contrast, expression of NTR1-Y06 in non-adapted cells (second column) allows visualization of functional receptor at the cell surface by ligand binding. These cells show also strong signals for mCh, however still to a large extent localized in the cell interior. Similar to wild-type NTR1, NTR1-Y06 expression in the non-adapted strain gives rise to non-expressing cells, depicting neither a signal for ligand binding nor for mCh. For expression of NTR1-Y06 in the adapted strain (third column), significantly fewer non-expressing cells are detected and expressing cells show at the surface a strong signal for both ligand-binding and mCh, with only little mCh detected in the cell interior. For a negative control (fourth column) cells expressing NTR1-Y06 without a mCh fusion were incubated with fluorescently labeled neurotensin in excess of non-labeled neurotensin. Representative pictures are shown.

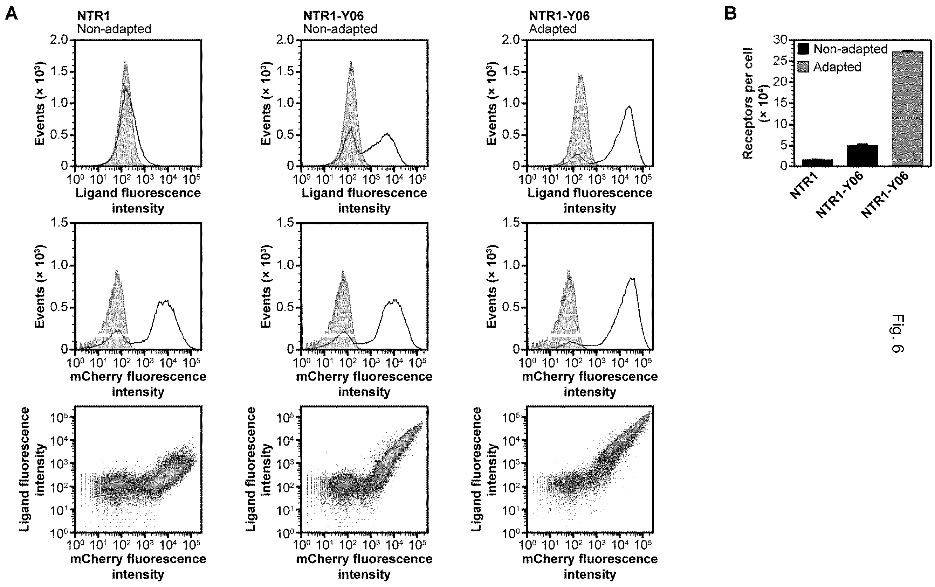

Fig. 6 shows FC and RLBA analysis of non-adapted and adapted strains expressing NTR1 variants with a C-terminal mCh fusion. (A) FC data of non-adapted strains expressing NTR1 (left panel), NTR1-Y06 (middle panel), and the adapted strain expressing NTR1-Y06 (right panel) are shown. In the histogram plots of ligand-binding experiments (top row), the surface expression of active receptor variants are compared with total signal (black curves) and nonspecific signal (gray, tinted) shown. NTR1 shows only little active receptor at the surface, whereas surface expression is significantly increased for NTR1-Y06 in the non-adapted and the adapted strain (shift of the specific signal towards higher fluorescence intensity). Compared to the non-adapted strain expressing NTR1-Y06 with a mCh fusion, adaptation further leads to a substantial decrease of the fraction of cells showing no surface expression of active receptor. In the histograms of mCh expression (middle row) total receptor produced is quantified. Black curves depict the mCh signal, whereas autofluorescence of cells expressing NTR1-Y06 without mCh-fusion incubated with fluorescent ligand represents the background (gray, tinted). NTR1 and NTR1-Y06 expressions in non-adapted strains have very similar mCh signal profiles. Since for expression of NTR1 in ligand-binding FC experiments only little active receptor at the surface is detected, the strong mCh signal means that most of NTR1 must be intracellularly retained. A subpopulation of cells not expressing any receptor at all is seen for all variants (note double peak), but this non-expressing fraction is significantly decreased in the adapted strain. In correlation analysis (bottom row) mCh fluorescence intensity (*total* receptor produced) is compared to ligand-binding fluorescence intensity (*functional* receptor at the surface). For expression of wild-type NTR1, functional receptor at the surface does not correlate well with total receptor produced. This correlation is better for NTR1-Y06 expressed in the non-adapted strain, and if NTR1-Y06 is expressed in the adapted strain, functional receptor at the surface correlates well with total receptor produced. *(B)* Measurement of average total functional receptors per cell by RLBA. Functional expression levels of receptors with a mCh-fusion increase from wild-type receptor to the evolved variant by a factor of three in the non-adapted strain. Compared to expression of NTR1-Y06 in the non-adapted strain, adaptation leads to a further increase in average total functional expression by a factor of 6. Non-specific signal was subtracted from total signal. Error bars indicate standard deviations from triplicates of a representative expression experiment.

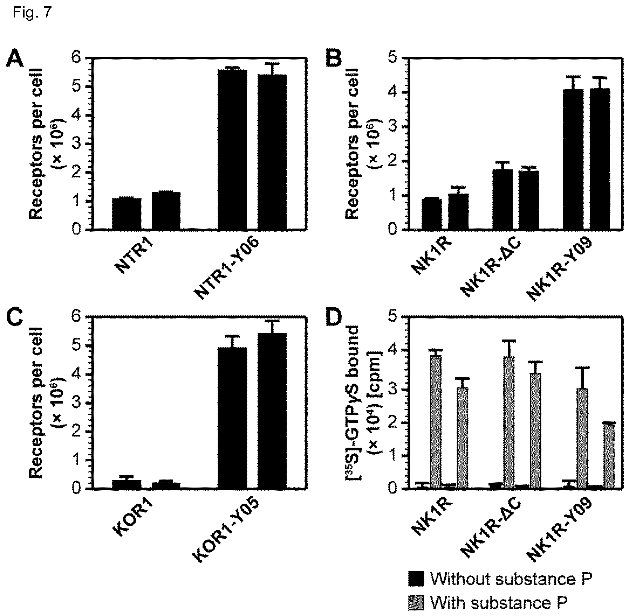

Fig. 7 shows functional expression levels of evolved GPCRs in *Sf*9 insect cells (for comparison reasons) and signaling activity of NK1R variants. *(A)*, *(B)*, *(C)* Measurement of average total functional receptors per cell by RLBA of NTR1, NK1R, and KOR1 variants. Compared to wild-type GPCRs all evolved variants show a significant increase of average receptor-per-cell levels. NTR1-Y06 shows a fivefold increase compared to wild-type NTR1, NK1R-Y09 shows a fourfold and twofold increase compared to NK1R and NK1R-ΔC, respectively, and the strongest increase (27-fold) is detected for KOR1-Y05 compared to wild-type KOR1. For each GPCR two independent expression experiments were performed (separate bars). Non-specific signal was subtracted from total signal. Error bars indicate standard deviations from triplicates. (*D)* Measurement of signaling activity of NK1R variants by [³⁵S]-GTPγS binding. Equal amounts of active GPCR and reconstituted G protein were assayed in the presence (grey) and absence (black) of substance P. All variants show a low basal activity without agonist stimulation. Upon addition of agonist, signaling is detected by [³⁵S]-GTPγS binding. NK1R and NK1R-ΔC have identical signaling activities, and signaling of NK1R-Y09 remains similar to wild-type receptor. For each GPCR variant two independent signaling assays from two independent expression experiments were performed (separate bars). Error bars indicate standard deviations from triplicates.

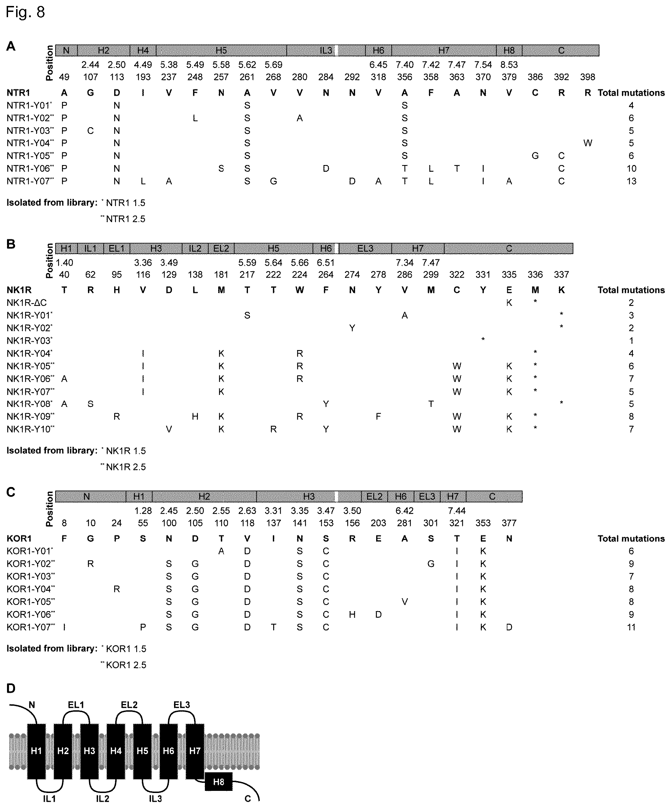

Fig. 8 shows an overview of the most highly enriched clones during evolution for each GPCR. Mutations of each clone are indicated and the corresponding wild-type amino acids are given on top of the mutation data. Positions of mutations are indicated by structural regions, Ballesteros-Weinstein numbering (3), and sequential amino acid numbering. *(A)* NTR1 variants. *(B)* NK1R variants. *(C)* KOR1 variants. *(D)* Scheme of GPCR topology depicting the different regions harboring mutations.

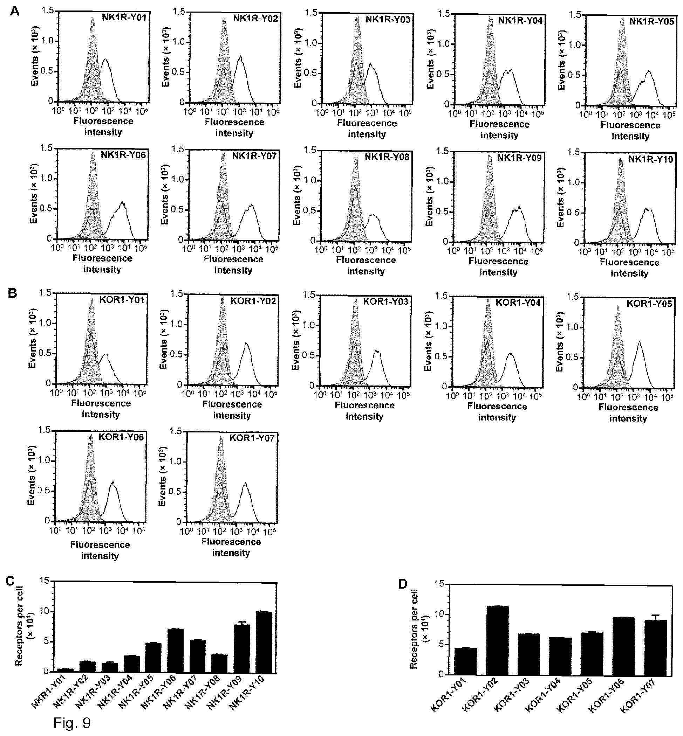

Fig. 9 shows functional expression of selected NK1R and KOR1 variants in non-adapted yeast strains. *(A), (B)* Histogram plots of ligand binding FC data of NK1R and KOR1 variants, respectively, with total signal (black curves) and nonspecific signal (gray, tinted) shown. Non-specific signal was obtained in the presence of an excess of unlabeled ligand. All variants show an increase in functional expression compared to the corresponding wild-type GPCRs (NK1R and KOR1, cf. Fig. 2 *C, E*). While the specific signal of the expressing cells of most variants is similar or slightly lower compared to the corresponding library pools (NK1R 2.5 and KOR1 2.5, cf. Fig. 2 *C*, *E*)*,* a higher fraction of cells not expressing any functional receptor at the surface is observed for the individual variants. *(C)*, *(D)* Measurement of average total functional receptors per cell by RLBA. While all variants show an increase in functional expression compared to the corresponding wild-type GPCRs, for most variants the average numbers of receptors per cell measured by RLBA are lower than for the corresponding library pools (cf. Fig. 2 *D*, *F*)*.* Non-specific signal was subtracted from total signal. Error bars indicate standard deviations from triplicates of a representative expression experiment.

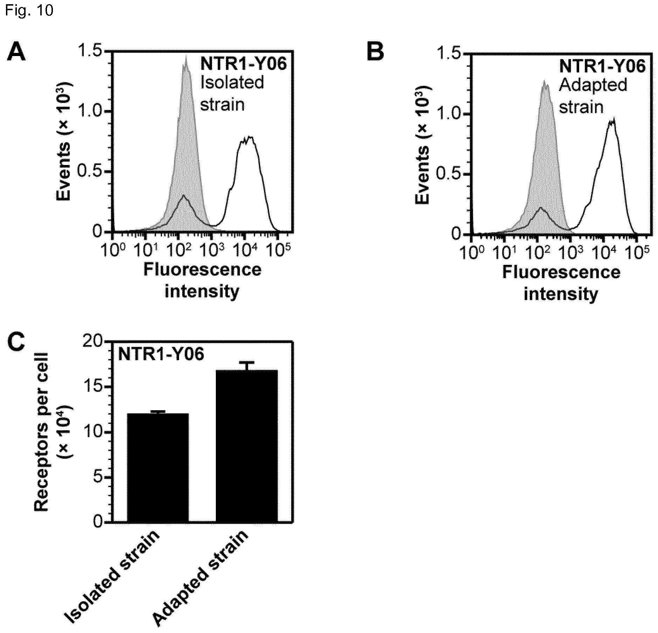

Fig. 10 shows functional expression levels of NTR1-Y06 in a yeast strain directly isolated from the NTR1 2.5 pool and a newly transformed strain subsequently adapted by phenotypic selection with FACS. *(A)*, *(B)* Histogram plots of ligand-binding FC data with total signal (black curves) and nonspecific signal (gray, tinted) are shown. Non-specific signal was obtained in the presence of an excess of unlabeled ligand. The isolated strain shows a similar expression profile as the NTR1 2.5 library pool (cf. Fig. 2 *A*), in contrast to the non-adapted retransformed strain expressing NTR1-Y06 (cf. Fig. 3 A). In the subsequently adapted strain shown in *(B)*, obtained after five rounds of phenotypic selection by FACS with the newly transformed strain, high surface expression levels are reconstituted. *(C)* Measurement of average total functional receptors per cell by RLBA. Compared to the retransformed strain expressing NTR1-Y06 (cf. Fig. 3 *B*), high functional NTR1-Y06 expression in the isolated strain as well as reconstitution of this phenotype in the adapted strain is detected. Non-specific signal was subtracted from total signal. Error bars indicate standard deviations from triplicates of a representative expression experiment.

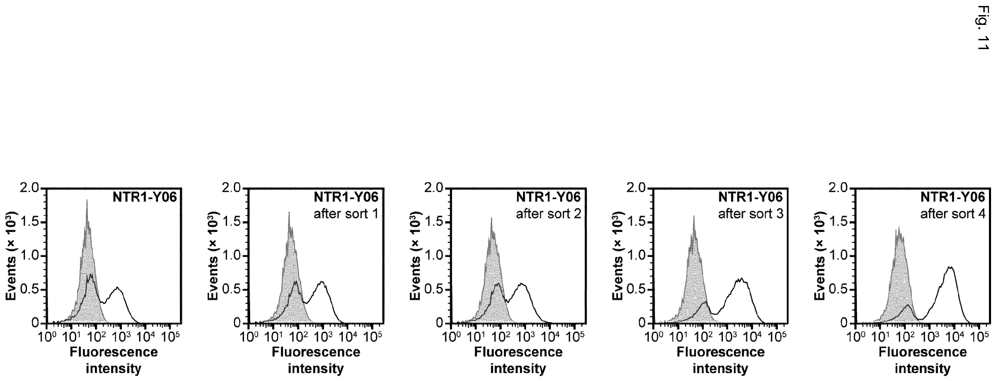

Fig. 11 shows expression profiles of NTR1-Y06 after individual sorts during phenotypic selection for host adaptation. Histogram plots of ligand binding FC data with total signal (black curves) and nonspecific signal (gray, tinted) are shown. Non-specific signal was obtained in the presence of an excess of unlabeled ligand. In the course of the phenotypic selection in which the cells with the highest NTR1-Y06 expression were sorted (gating of the top 1% of the most fluorescent cells), the population of cells not expressing any functional receptor at the surface gradually decreases. At the same time, the specific signal of the expressing cells shifts to higher levels during the selection, indicating an increase of functional receptors per cell at the surface of these cells. Note that two sorts are required to induce the adaptation. While the expression profile is not significantly changed after the first two sorts, a clear decrease of the subpopulation with no surface expression of active NTR1-Y06 as well as a shift of the specific signal within the expressing subpopulation is observed after sort 3. This trend is continued in subsequent sorts, as seen in the expression profile after sort 4.

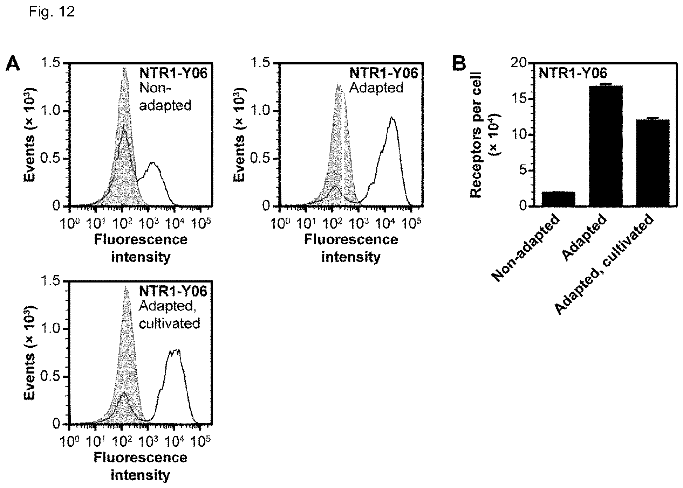

Fig. 12 shows functional expression of NTR1-Y06 in non-adapted, adapted, and adapted strain previously cultivated under non-expressing conditions in *S. cerevisiae. (A)* Histogram plots of ligand binding FC data with total signal (black curves) and nonspecific signal (gray, tinted) are shown. Non-specific signal was obtained in the presence of an excess of unlabeled ligand. Adaptation of newly transformed strain expressing NTR1-Y06 leads to a higher specific signal (receptors per cell) of the expressing cell subpopulation and a decrease of the subpopulation not showing any surface expression of active receptor. If the adapted strain is repetitively cultivated under non-expressing conditions, the average expression level decreases again, depicted by a drop of the specific signal (receptors per cell) in the expressing subpopulation and an increase of the fraction of cells with no surface expression of active NTR1-Y06. *(B)* Measurement of average total functional receptors per cell by RLBA. Results from RLBA show the significant increase of the number of average receptors per cell from the non-adapted to the adapted strain, which in turn drops again by about 30% if the adapted strain is repetitively cultivated under non-expressing conditions prior to inducing expression. The lack of complete reversion to the original lower expression level of the non-adapted strain may be attributed to leaky expression, sufficient to partially retain the high-expression phenotype even under non-inducing conditions. Non-specific signal was subtracted from total signal. Error bars indicate standard deviations from triplicates of a representative expression experiment.

Fig. 13 shows functional expression of NK1R-Y09 and KOR1-Y05 in non-adapted and adapted *S. cerevisiae* strain. *(A)*, *(C)* Histogram plots of ligand binding FC data with total signal (red curves) and nonspecific signal (green, tinted) are shown. Non-specific signal was obtained in the presence of an excess of unlabeled ligand. While the specific expression signals (receptors per cell) of expressing cells in the adapted strains slightly increase compared to the non-adapted strains, a significant decrease of the subpopulation with no surface expression of active receptor is observed. *(B)*, *(D)* Measurement of average total functional receptors per cell by RLBA. The decrease of the fraction of cells which show no surface expression of active receptor in the adapted strains, shown in the FC data, leads to an increase of the average functional expression measured with RLBA. Non-specific signal was subtracted from total signal. Error bars indicate standard deviations from triplicates of a representative expression experiment.

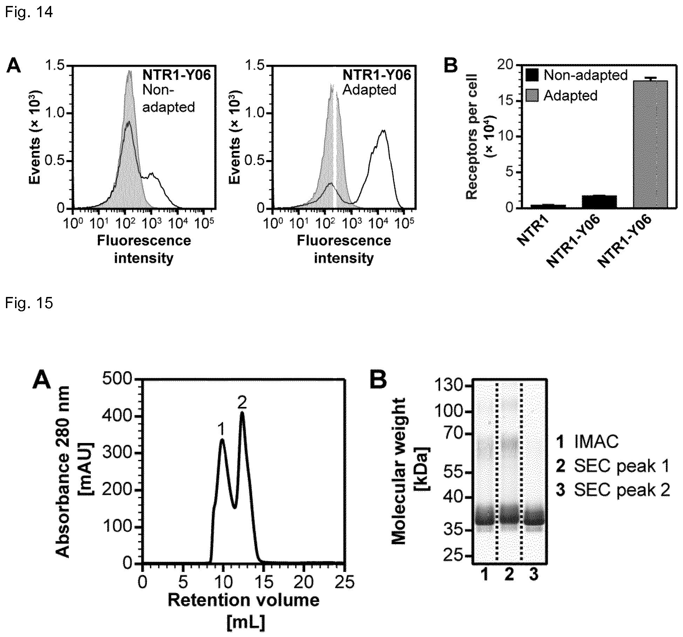

Fig. 14 shows functional expression of HA-tagged NTR1-Y06 in non-adapted and adapted *S. cerevisiae* strain. *(A)* Histogram plots of ligand-binding FC data with total signal (black curves) and nonspecific signal (gray, tinted) are shown. Non-specific signal was obtained in the presence of an excess of unlabeled ligand. Adaptation of the strain expressing NTR1-Y06 with a C-terminal HA-tag leads to a decrease of the fraction of cells showing no surface expression of active receptor and an increase of active receptors per cell at the surface of cells with surface expression (shift of specific signal towards higher fluorescence intensity). *(B)* Measurement of average total functional receptors per cell by RLBA. Functional expression levels increase from wild-type receptor to the evolved variant by a factor of four in the non-adapted strain. Compared to expression of HA-tagged NTR1-Y06 in the non-adapted strain, adaptation leads to a further increase in average total functional expression by a factor of 10. Non-specific signal was subtracted from total signal. Error bars indicate standard deviations from triplicates of a representative expression experiment.

Fig. 15 shows purification of NK1R-Y09. *(A)* SEC profile of purified receptor after IMAC. Two peaks are obtained of which peak 1 most likely corresponds to defined higher oligomeric states of NK1R-Y09, while peak 2 represents the monomeric receptor fraction. *(B)* Analysis of purified NK1R-Y09 by SDS-PAGE under reducing conditions. Equal amounts of total protein were loaded in each lane. After IMAC (lane 1) pure NK1R-Y09 (38.5 kDa) is obtained, with detection of weak additional protein bands at higher molecular weight. Such bands represent most likely oligomers of NK1R-Y09, which are not disintegrated under conditions used. Next to a strong band for monomeric NK1R-Y09, the bands of higher molecular weight are also detected in the fraction of peak 1 (lane 2), while in the fraction of peak 2 (lane 3) monomeric NK1R-Y09 is the sole species. Note that NK1R-Y09 was not further engineered and expressed in insect cells as it was obtained from the selection in yeast. For purifications with the aim to perform crystallization trials, some further engineering will be required, for instance removal of potential glycosylation sites, N-terminal truncations, loop deletions, and/or introduction of fusion proteins (e.g. T4 lysozyme or thermostabilized apocytochrome b₅₆₂RIL (Chun et al. (2012), Structure 20:967-976)). Such measures potentially further improve purification of the receptor by reducing heterogeneity, restricting conformational flexibility, and increasing stability (Maeda & Schertler (2013), Curr Opin Struct Biol 23:381-392).

### Examples

### Example 1: Generation of high-expressing functional GPCRs by directed evolution in yeast (parts relating to insect cells are for comparison reasons)

With about 800 different members in the human genome, G protein-coupled receptors (GPCRs) comprise the largest superfamily of cell surface receptors. GPCRs evolved to highly versatile signaling mediators in eukaryotic life, responding to a wide variety of ligands and transducing signals via heterotrimeric G proteins as well as in a G protein-independent fashion. The pivotal role of GPCRs is reflected by the great number of human diseases linked to aberrant GPCR signaling, for instance obesity, diabetes, cardiovascular diseases, osteoporosis, immunological disorders, neurodegenerative diseases, and cancer. As a consequence, GPCRs represent highly relevant drug targets for the pharmaceutical industry. About 30 - 50% of marketed drugs act on GPCRs or GPCR-associated mechanisms, among them many top-selling drugs.

The major challenges in structural and detailed biochemical investigation of GPCRs are the difficulties to produce the desired receptors in sufficient amounts as well as their inherent instability and flexibility. Advances in expression and crystallization strategies, like the use of the baculovirus/insect cell expression system, or the establishment of integral membrane protein crystallization in lipidic cubic phases (LCP), lead to breakthroughs in determination of three-dimensional receptor structures by X-ray crystallography. While the available structural datasets provided the scientific community with insights towards the mechanisms of GPCR function at the atomic level and might begin to enable rational structure-based drug design, it is clear that detailed understanding of receptor dynamics and conformational changes has still remained rather incomplete.

Furthermore, the 26 unique GPCR structures deposited in the Protein Data Bank (PDB, http://www.pdb.org) to date still represent only a minor fraction of all receptors (< 4%), reflecting that the bottlenecks for structural investigations of GPCRs persist. For instance, while insect cells were successfully used for production of recombinant receptors for about 85% of all GPCR structures determined so far (source: PDB; excluding structures obtained from protein extracted from native tissues), this expression system does not provide a generic solution. Even in insect cells several members of the GPCR superfamily are expressed at low yields, suggesting fundamental issues with the biosynthesis and membrane insertion of these receptors also in eukaryotic cells. This problem cannot be solved by simple optimization of expression conditions, especially since there seems to be no consistency of optimal conditions for individual GPCRs. Thus, to facilitate and accelerate GPCR research, novel approaches are required.

Recently, the inventors developed a method to increase functional expression levels of GPCRs in *Escherichia coli* by directed evolution (Sarkar et al. (2008), Proc Natl Acad Sci USA 105:14808-14813; Dodevski & Plückthun (2011), J Mol Biol 408:599-615). Random mutagenesis of wild-type GPCRs followed by selection with fluorescence-activated cell sorting (FACS) allowed isolation of high-expression variants from randomized libraries of four different GPCRs, namely neurotensin receptor 1 (NTR1), NK-1 receptor (NK1R, also termed tachykinin receptor 1 or substance-P receptor) and of the alpha-1A and alpha-1B adrenergic receptors. In successive studies for NTR1, the obtained first-generation variants built the basis for creation of second-generation mutants by extensive selection (Schlinkmann et al. (2012), Proc Natl Acad Sci USA 109:9810-9815) and exhaustive recombination (Schlinkmann et al. (2012), J Mol Biol 422:414-428). The synthetic libraries created in these studies were also used to generate NTR1 variants with improved stability in short-chain detergents by a complementary directed evolution approach termed CHESS (Scott & Plückthun (2013), J Mol Biol 425:662-677; Scott et al. (2014), Biochim Biophys Acta 1838:2817-2824). Ultimately, these efforts resulted in the structures of three different variants of agonist-bound NTR1 from material produced in *E. coli* (Egloff et al. (2014), Proc Natl Acad Sci USA 111:E655-62), underlining the power of directed evolution in membrane protein engineering.

The successful results obtained with evolution in *E. coli* and the proven effectiveness of eukaryotic expression hosts demanded to further develop this approach towards high functional GPCR expression specifically in eukaryotic expression systems. The inventors hypothesized that it might be advantageous to perform evolution of GPCRs directly in a eukaryotic system in order to achieve specific sequence adaptation and thereby improved functional production in eukaryotes. Furthermore, compared to eukaryotic hosts, *E. coli* is not able to perform post-translational modifications, lacks the secretory quality control and translocation machinery for eukaryotic membrane proteins, and differs in membrane composition. Therefore, directed evolution of GPCRs in *E. coli* is limited to receptors which inherently express in a prokaryotic system above the threshold required for successful evolution, thus not having strict requirements for eukaryotic processing or membrane composition.

Here the inventors disclose the establishment of a fast, efficient and robust method to increase functional expression of GPCRs in eukaryotic hosts by directed evolution in the yeast *Saccharomyces cerevisiae.* The method is generally applicable as demonstrated with three different GPCRs, leading, with only two rounds of evolution, to receptor variants which show high functional expression in both yeast and insect cells. In addition, functional expression levels in yeast can be further increased reproducibly by induced host adaptation.

### Directed evolution of three different GPCRs in yeast results in high functional expression in S. cerevisiae

The general approach of this method is depicted in Fig. 1. First, the wild-type GPCR gene is randomized by error-prone PCR and the resulting DNA library is used for transformation of yeast. The insert DNA and yeast expression vector backbone are assembled *in vivo* via designed homologous recombination sites. Next, expression in the obtained yeast library is induced, and subsequently cells are treated with an optimized buffer for permeabilization of the yeast cell wall. Permeabilization is necessary for allowing access and thus binding of administered fluorescent ligand to the functionally expressed receptors in the plasma membrane. After incubation with saturating concentrations of fluorescent ligand, unbound ligand is removed by washing, and cells are subjected to selection with FACS. Yeast cells expressing variants with the largest number of functional receptor molecules at the surface correspondingly exhibit the highest fluorescence. These cells are isolated by gating the top 0.5 - 1% of the most fluorescent yeast cells, and sorting them directly into growth medium for subsequent propagation. In order to achieve strong enrichment of cells expressing GPCR variants with the desired phenotype, several repetitive cycles of expression, incubation with fluorescent ligand, and FACS are performed. Plasmid DNA can be isolated from selected cells after FACS for analysis or introduction of further diversity by additional random mutagenesis, thereby starting the next round of evolution.

The inventors aimed to evolve three different GPCRs in parallel: (i) rat NTR1, (ii) human NK1R, and (iii) human kappa-type opioid receptor (KOR1). NTR1 has been shown to be readily evolvable in the *E. coli*-based system in several studies, and thus was considered a positive control. NK1R has been successfully subjected to directed evolution towards higher expression in *E. coli* as well (Dodevski & Plückthun (2011), J Mol Biol 408:599-615). However, despite strong relative improvements, due to the very low expression levels of wild-type NK1R in the prokaryote, the evolved receptor variants expressed still at only moderate absolute levels compared to the other receptors evolved in *E. coli* (Sarkar et al. (2008), Proc Natl Acad Sci USA 105:14808-14813; Dodevski & Plückthun (2011), J Mol Biol 408:599-615). Moreover, expression levels of these evolved NK1R variants were also low in *S. cerevisiae* (see below), suggesting that further improvement of functional production might be possible, which may benefit future studies on this receptor. Concerning KOR1, no attempts to evolve this receptor have been undertaken so far and it represents a challenging example regarding heterologous expression.

Following the procedure outlined above, for each of the three receptors only two rounds of evolution - each round consisting of one randomization by error-prone PCR and five subsequent selections by FACS - were sufficient to strongly increase functional expression levels. Fig. 2 shows a comparison of the functional expression levels in *S. cerevisiae* of wild-type GPCRs, NTR1 and NK1R variants previously evolved in the *E. coli*-based system (NTR1-D03 and NK1R-E11, respectively), and the library pools obtained after the second round of evolution, namely NTR1 2.5, NK1R 2.5, and KOR1 2.5 (library nomenclature: GPCR a.b, where a is the number of total randomizations, and b is the number of total FACS selections). Functional expression levels were measured either with flow cytometry (FC) or radioligand binding assays (RLBAs). Note that FC ligand-binding experiments determine functional receptors exclusively in the plasma membrane at the surface of intact individual cells, whereas RLBAs account for the total amount of functional receptors averaged across an entire population of lysed cells, and thus also detect functional GPCRs in intracellular membranes. For the wild-type GPCRs no specific signal is obtained in FC experiments, thus no active receptor in the plasma membrane at the surface is detected (Fig. 2 *A, C*, *E*). By contrast, in RLBAs for NTR1 and KOR1 low expression levels of functional receptor are measured, indicating that a small amount of active receptor is present, however, most likely retained in intracellular membranes (Fig. 2 *B*, *F*)*.* For NK1R, no functional receptor is detected in any of the methods used (Fig. 2 *C*, *D*), consistent with what has been reported before (Butz et al. (2003), Biotechnol Bioeng 84:292-304).

Strikingly, the expression levels of the selected libraries are not only much higher than the wild-type receptors but also higher than the corresponding variants evolved in *E. coli* (NTR1-D03, NK1R-E11), illustrated by FC and RLBA analysis. 100,000 - 150,000 total functional receptors per cell are measured in RLBA for the selected yeast libraries, representing a 25 - 50-fold and 5 - 20-fold increase compared to the corresponding wild-type GPCRs and *E. coli*-evolved variants, respectively (Fig. 2 *B*, *D*, *F*)*.* The FC data confirm active receptor at the surface by showing a distinct specific signal for both evolved variants from *E. coli* and selected yeast libraries (Fig. 2 *A, C*, *E*). However, the specific signal of the selected yeast libraries is shifted towards higher fluorescence intensity compared to the *E. coli*-evolved variants, reflecting an increase in active receptors at the surface. Furthermore, a significantly smaller subpopulation of cells not expressing any functional receptor at the surface is detected in the selected yeast libraries.

Such a division into two subpopulations was reproducibly observed in FC experiments upon expression of all GPCRs tested in both yeast single clones and libraries. Hence, this effect appears to be an intrinsic feature of GPCR expression in *S. cerevisiae,* in which functional expression at the surface is lacking in a substantial fraction of cells. For instance, depicted by the FC data of the *E. coli*-evolved variants, active surface expression of NTR1-D03 is obtained in about 50% of all cells, and for the low-expressing variant NKR1-E11 only a minority of cells show active surface expression (Fig. 2 *A, C*). It remains unclear why some cells of the population grown from one single clone do not produce any functional receptor at the surface, but a loss of the expression vector in these cells can be excluded since all cultivations are performed exclusively in selective minimal medium.

### Improved expression levels of enriched receptor variants in yeast are further increased by host adaptation occurring concurrently with selection.

In order to identify enriched clones, plasmid DNA was isolated from the library pools after the first (stage 1.5 libraries) and second round (stage 2.5 libraries) of evolution for DNA sequencing. A summary of the selected clones depicting the different mutations of each clone is given in Fig. 8. Interestingly, whereas for NTR1 and KOR1 only full-length variants were selected, all selected NK1R variants contain a stop codon at a position within a narrow range of seven amino acids after the presumed helix 8 and palmitoylation site, resulting in a shortened C-terminus. This indicates that a full-length C-terminus is strongly selected against and a truncation leads to a significant increase in expression for this receptor. Since no functional wild-type NK1R can be detected in yeast, the inventors constructed an alternative reference variant, termed NK1R-ΔC, with the most prominently selected C-terminal truncation combined with the wild-type sequence. This provided the inventors with a reference for the selected mutations and also allowed them to quantify how much a C-terminal truncation contributes to improved functional expression for NK1R.

Individual clones were analyzed after retransformation and revealed that all enriched receptor variants were indeed significantly better expressed in yeast than the corresponding wild-type receptors (Fig. 3 and Fig. 9). This indicates that variants have indeed been obtained that are better adapted to the biosynthesis in this eukaryotic host.

While only a small fraction of cells not expressing any functional receptor at the surface was detected in the selected GPCR libraries (Fig. 2 *A, C*, *E*), a larger subpopulation of such cells was observed when the individual variants had been retransformed into fresh host cells (Fig. 3 *A* and Fig. 9 *A*, *B*)*.* Furthermore, lower receptor-per-cell levels of individual NTR1 variants in cells with active surface expression after retransformation were also detected, as indicated by the shift of the specific FC signal towards lower fluorescence intensity (Fig. 3 *A*)*.* Consistent with these observations of a combined effect in FC, significantly lower average RLBA readings were obtained in retransformed strains expressing individual NTR1 variants (Fig. 3 *B*), compared to the selected GPCR library (Fig. 2 *B*)*.*

The inventors hypothesized that in the course of selection an adaptation of the expression host occurred which led to a further increased level of membrane protein expression in the selected libraries. To test this hypothesis, a yeast clone, expressing a strongly selected variant termed NTR1-Y06, was directly isolated from the NTR1 2.5 library pool. As expected, the isolated strain expresses NTR1-Y06 at high functional levels, with a similar expression profile as the NTR1 2.5 library pool (Fig. 10 *A, C*). Sequencing of whole plasmid DNA isolated from both the retransformed and the isolated strain expressing NTR1-Y06 revealed no difference. Thus, the inventors concluded that the actual procedure of repetitive expression and sorting of the best expressing clones by FACS induced an adaptation of yeast towards improved GPCR production. To test this hypothesis, five subsequent mock selections (without any randomization of the sequence) with FACS with the retransformed single clone expressing NTR1-Y06 were performed, in which the cells with the highest NTR1-Y06 expression were isolated by gating the top 1.0% of the most fluorescent cells. Indeed, in the course of this phenotypic selections the strain adapted to higher expression of NTR1-Y06 by a gradual decrease of cells with no active surface expression and an increase of functional receptors per cell in the subpopulation with surface expression (Fig. 11). Finally after adaptation, NTR1-Y06 is expressed at total functional levels as high as the expression levels obtained in the selected NTR1 2.5 pool with about 160,000 average functional receptors per cell and a similar expression profile (Fig. 10 *B*, *C*). However, by repetitive cultivation of the adapted strain under non-expressing conditions, a partial reversion of the adaptive effect by a 30% drop of the expression level was observed (Fig. 12).

In order to test whether the phenotypic adaptation might even have been the main feature of the selection, the inventors also tried to adapt the strain expressing wild-type NTR1. However, after FACS, the sorted cells did not propagate anymore, preventing repetitive selections. This observation clearly shows that the selection of mutations, conferring improved properties to the receptors, is a strict prerequisite of the host adaptation, and that the sequence change is actually the key evolutionary event. The failure to induce adaptation in cells expressing wild-type NTR1 may be explained by the combined stressful effects of expression of the toxic wild-type GPCR followed by selection, together limiting cell survival.

Hence, adaptation induced by repetitive sorting appears to be restricted to evolved GPCR variants, which intrinsically are less toxic for the cells. Indeed, adaptation of strains expressing NK1R-Y09 and KOR1-Y05, evolved NK1R and KOR1 variants, respectively, was possible as well. Both variants were strongly enriched during selection and newly transformed cells expressing NK1R-Y09 or KOR1-Y05 showed lower expression levels than the corresponding library pools. For each of the variants five rounds of phenotypic selection by FACS, identical to the procedure used to adapt the strain expressing NTR1-Y06, were performed. For both variants host adaptation towards higher expression levels was observed, gradually arising in the course of selection (Fig. 13). Even though the host adaptation effect for NK1R-Y09 and KOR1-Y05 is less pronounced than for NTR1-Y06, these results suggest that the adaptation is not receptor-specific, and can be reproducibly induced, if evolved receptors are expressed.

### The fraction of functional receptor is increased in adapted strains, while the amount of total receptor produced is not significantly higher.

Since host adaptation was most pronounced for expression of NTR1-Y06, this variant was used to further study the effect. While ligand-binding FC analysis and RLBA permit the detection of active receptor, neither of these methods allow quantification of total receptor produced regardless of ligand-binding activity. Therefore, to detect total receptor protein produced in different strains by immunoblotting, NTR1 and NTR1-Y06 expression constructs with a hemagglutinin (HA) tag at the C-terminus of the receptors were created.

The strain expressing the HA-tagged NTR1-Y06 was adapted towards higher expression by five rounds of phenotypic selection with FACS (Fig. 14 A). Subsequently, total receptor production for the different strains expressing wild-type NTR1 and NTR1-Y06, the latter in non-adapted and adapted strain, was measured by HA tag detection in quantitative Western blots from whole cell lysates, using equal numbers of yeast cells after expression (Fig. 4).

In non-adapted strains, the amount of *total* receptor produced increases from wild-type NTR1 to the evolved variants by a factor of two (Fig. 4). The observed increase of *functional* expression by RLBA data is by a factor of four (Fig. 14 *B*). This indicates that the ratio of functional receptor to total receptor produced is just slightly increased from wild-type to the evolved receptor.

However, when comparing the adapted to the non-adapted strain, based on RLBA data, there is a tenfold increase in functional expression of NTR1-Y06 (Fig. 14 *B*) but there is less than a twofold increase of total receptor production (Fig. 4). Since the amount of total receptor produced increases only slightly, it can be deduced that in the adapted strain the fraction of functional receptor is significantly increased.

### Adaptation increases surface expression of active receptor, reduces the number of cells exclusively expressing intracellularly retained GPCRs, and reduces the non-expressing cell subpopulation.

To further describe the host adaptation effect by assessing cellular localization and functionality of NTR1 variants in non-adapted and adapted strains, NTR1 and NTR1-Y06 constructs with a C-terminal mCherry (mCh) fusion were used for confocal microscopy studies and FC experiments: while total receptor is quantified by mCh, only functional receptors located at the cell surface in the plasma membrane will exhibit a signal from fluorescent ligand binding.

Identical to the HA-tagged variant, the strain expressing NTR1-Y06 with a mCh fusion was first adapted by phenotypic selection. Next, the non-adapted strains expressing NTR1 and NTR1-Y06, as well as the adapted strain expressing NTR1-Y06 were exposed to fluorescent ligand to perform co-localization studies with confocal fluorescence microscopy (Fig. 5).

As seen in ligand-binding FC experiments (Fig. 6 *A*), levels of functional wild-type NTR1 in the plasma membrane are very low, and consequently no ligand-binding signal is detected in microscopy (Fig. 5). In contrast, clear signals of mCh are obtained for some cells, however exclusively located in the cell interior, thus reflecting cells expressing intracellularly retained NTR1-mCh fusion receptors. According to the low RLBA readings obtained for wild-type NTR1 (Fig. 6 *B*), most of the intracellular receptor must be inactive. Additionally, many other cells are detected without any mCh signal at all, representing a non-expressing subpopulation (Fig. 5). Notably, both of these subpopulations, cells exclusively expressing intracellularly retained receptor as well as non-expressing cells, cannot be discriminated in ligand-binding experiments with FC. Thus, these two subpopulations together constitute the fraction of cells not showing any surface expression in FC ligand-binding data.

Evolved NTR1-Y06 expressed in the non-adapted strain shows detectable ligand binding at the surface as well as a mCh signal (Fig. 5). However, the signal of mCh at the plasma membrane is still rather weak with a strong mCh signal detected in the cell interior, indicating that, similar to wild-type NTR1, a substantial amount of receptor is retained in intracellular membranes. Furthermore, non-expressing cells without any signal, neither for ligand binding nor for mCh, are frequently detected as well. Hence, also for the evolved receptor, the subpopulation of cells with no surface expression detected in FC ligand-binding experiments comprises two types of cells: those without any receptor expression, and those cells in which expressed receptor is not translocated to the plasma membrane.

The situation is different for NTR1-Y06 expressed in the adapted strain, in which strong signals for ligand binding and mCh are detected in the plasma membrane (Fig. 5). Even though some signal is still seen in the cell interior, mCh is mainly localized at the surface. Furthermore, significantly fewer non-expressing cells are observed.

Further investigations by FC experiments (Fig. 6 A), comparing the ligand-binding to the mCh signal, confirmed the results obtained in the confocal microscopy studies: signals for mCh in the expressing subpopulation are detected for all variants, however, for wild-type NTR1 the mCh fluorescence intensity does not correlate well with ligand-binding signal intensity. This is reflecting the fact that most of the wild-type receptor is not translocating to the cell surface. The finding that fluorescence intensity of wild-type GPCRs fused to an autofluorescent protein (e.g. GFP or mCh) correlates only weakly with functional receptor levels has been described before. Interestingly, this correlation is better for NTR1-Y06 expression in the non-adapted strain, and for NTR1-Y06 expressed in the adapted strain, total receptor produced and functional receptor correlate well. In this case, the receptor is mostly transported to the cell surface, reflecting the decrease of cells with exclusively intracellular receptor expression. Interestingly, the subpopulation showing not any mCh signal (non-expressing cells) is also decreased in the adapted strain, compared to the non-adapted strains, further leading to an overall increase of functional GPCR production levels in the adapted strain.

In summary, host adaptation of *S. cerevisiae* for GPCR expression increases surface expression of active receptor without increasing the total amount of receptor produced, which is equivalent to a higher percentage of active receptor produced. In contrast to non-adapted cells, for which a large fraction of cells does not show any functional surface expression, adapted cells show only small amounts of intracellularly retained receptor. Since a significant part of intracellular receptor is inactive, host adaptation leads to an overall increase of active receptor. Additionally, the subpopulation of cells not expressing any receptor at all is also decreased in the adapted strains compared to the non-adapted ones, contributing further to higher average functional production levels.

### Evolved variants of all three GPCRs show high functional expression levels in Spodoptera frugiperda (Sf9) insect cells (comparative example).

An aspect described herein was to increase functional GPCR expression not only in yeast but in insect cells as well. Thus, the functional expression levels of the evolved GPCRs NTR1-Y06, NK1R-Y09, and KOR1-Y05 were determined in *Sf*9 insect cells and compared to the corresponding wild-type receptors. For all GPCRs identical standard expression conditions (non-optimized) were used. Indeed, a significant increase of functional expression of the evolved variants was observed. For NTR1-Y06 compared to wild-type NTR1, functional expression is increased 5-fold (Fig. 7 A), for NK1R-Y09 a 4-fold increase is measured over wild type (Fig. 7 B), and for KOR1-Y05 - notably being the most challenging example regarding expression studied in this work - a 27-fold increase of functional expression is detected (Fig. 7 C). The measured average functional receptor-per-cell levels (4.1 × 10⁶ - 5.5 × 10⁶ receptors per cell) are high and surpass expression levels of first- and even second-generation NTR1 mutants generated in the *E. coli*-based directed evolution system when those are expressed in *Sf*9 insect cells (Schlinkmann et al. (2012), J Mol Biol 422:414-428).

With such high expression levels, as obtained for all of our three GPCR mutants tested, expression cultures in liter scales are sufficient to produce enough material for structural investigations. To test this, purifications of NK1R-Y09 from insect cell expression cultures were performed, reproducibly yielding 4 - 6 mg/L pure protein. Fig. 15 shows the analysis of purified NK1R-Y09 by size-exclusion (SEC) and SDS-PAGE.

Given the very high purification yields, NK1R-Y09 represents an interesting and promising candidate for structural investigations. Thus, the functionality of NK1R-Y09 was characterized by signaling assays based on [³⁵S]-GTPγS binding (Fig. 7 *D*)*.* NK1R-Y09 shows similar activity as wild-type NK1R and the C-terminally truncated variant NK1R-ΔC, both regarding [³⁵S]-GTPγS binding without stimulation (basal activity) and upon agonist stimulation, further underlining the potential of our approach.

### Summary

Production of recombinant GPCRs in functional form still remains a demanding, laborious, and cost-intensive task. Given that most of the recently determined three-dimensional GPCR structures were obtained from receptors produced in eukaryotic expression hosts and encouraged by the success of protein engineering methods for increased expression of GPCRs in *E. coli,* the inventors aimed to transfer the directed evolution approach to *S. cerevisiae* to specifically improve functional GPCR production in eukaryotes.

The yeast *S*. *cerevisiae*, characterized by fast growth, cost-effective cultivation, and ease of genetic manipulations, is an ideal eukaryotic host for protein engineering and directed evolution for several additional reasons: First, yeast surface display has become a very powerful technology in a range of different applications. Second, high-diversity libraries are nowadays easily obtained with yeast. Third, the yeast cell is equipped with the cellular machinery required for functional GPCR production, since *S. cerevisiae* endogenously expresses two different GPCRs with signaling pathways similar to higher eukaryotes. Fourth, yeast has already been extensively used for heterologous expression of several different GPCRs. And fifth, based on a widely used GPCR assay which couples heterologous GPCRs to the yeast endogenous signaling pathway, directed evolution approaches on GPCRs have been successfully used before with the aim to generate designer receptors exclusively activated by designer drugs (DREADDs) activated by new ligands (Armbruster et al. (2007), Proc Natl Acad Sci USA 104:5163-5168).

So far, functional production yields of GPCRs in yeast have generally remained low (Sarramegna et al. (2003), Cell Mol Life Sci 60:1529-1546). Strategies to increase GPCR expression in *S. cerevisiae* included optimization of expression conditions, co-expression of molecular chaperones, screening for high-expressing host clones, screening of engineered GPCR variants, or host engineering. In this specification the inventors disclose the general applicability of this invention to directly obtain GPCR variants with increased functional GPCR expression levels by evolving three different GPCRs. Remarkably, only two rounds of evolution - meaning two randomizations each followed by selection with FACS - were required to obtain highly increased expression levels, and thus it was possible to evolve all three GPCRs efficiently in a short time. In the *E. coli*-based system, usually four rounds of evolution were required. Furthermore, the inventors disclose that the yeast host cell can be adapted towards increased GPCR production. This is an inherent feature of the selection system, which happens concurrently with the selection of improved receptor variants. It is important to reiterate that the directed evolution *did* lead to sequence changes, whose improved expression phenotype could be transferred to insect cells, and that the host adaptation is only an additional factor occurring in yeast during the selection. Moreover, the inventors found that expression of an evolved receptor is a prerequisite for inducing the host adaptation, as attempts to induce host adaptation in cells expressing a wild-type GPCR failed. The host adaptation of yeast can reproducibly be induced by repetitive phenotypic selection with FACS. This usually requires 4 - 5 sorts, and the effect gradually increases during the selection.

Adapted strains show a significant increase of surface-expressed active receptor, leading to a much higher fraction of *functional* receptors in the plasma membrane, with only a small increase of *total* receptor produced. On the contrary, in non-adapted strains, and especially for expression of wild-type receptors, a large amount of receptors remain in intracellular compartments, of which a significant amount represents inactive misfolded protein. The overall higher average functional production yields in expression cultures of adapted cells can be explained by a decrease of the subpopulation of cells expressing intracellularly retained inactive receptor as well as a decrease of the non-expressing subpopulation.

In a comparative aspect, it was explored whether the obtained GPCR variants would permit higher expression in other eukaryotic hosts as well. Indeed, functional expression of GPCR variants evolved in yeast was also increased in insect cells. Notably, variants evolved in the *E. coli*-based system show less improved functional expression when moved to eukaryotic hosts like insect cells, compared to the variants generated in yeast (Schlinkmann et al. (2012), J Mol Biol 422:414-428). The high expression levels in *Sf*9 cells obtained with the novel yeast-evolved variants are sufficient for crystallographic investigations with expression cultures in liter scales under standard conditions. More specifically, purification of the NK1R variant NK1R-Y09 yielded very large quantities of pure protein. Furthermore, NK1R-Y09 showed signaling activities similar to wild-type NK1R, illustrating that the disclosed method can give rise to biologically active variants able to perform their naturally intended function, which consequently can be studied conveniently with these variants.

In summary, the method for directed evolution of GPCRs in *S. cerevisiae* disclosed herein allows efficient generation of GPCR variants with high functional expression in eukaryotic expression hosts in a short time. Using *S. cerevisiae* as evolution host resulted in GPCR variants with high functional expression levels in both yeast and insect cells, in the latter system by up to a factor 27. Thus, the mutations are transferable and selected variants showed a greater increase in expression in yeast as well as in insect cells than when evolution had been performed in *E. coli.*

The transferability of the beneficial effects of the receptor mutations to insect cells further promotes *Sf*9 cells as expression host for large-scale production of the generated mutants. Nonetheless, the fact that high expression levels in *S. cerevisiae* can now also be obtained as a result of GPCR evolution, combined with induced host adaptation, implies a potential of yeast as a large-scale production host. From a practical point of view, the fact that in adapted strains the ratio of functional receptor to total receptor produced is improved is a great advantage for purification strategies like IMAC, which cannot discriminate between non-functional and functional protein. This is of special interest for purifications of receptors for which no ligand affinity column, for instance like the cleavable ligand column as established for purification of NTR1 (Egloff et al. (2014), Protein Expr Purif, in press), is available.

The versatility of *S. cerevisiae* broadens the portfolio of GPCRs amenable to our approach, allowing evolution of GPCRs for which the previously established system in *E. coli* is inadequate. For instance, for GPCRs like KOR1, which show very low functional expression levels even in eukaryotes, expression levels in *E. coli* may be below the threshold needed for successful evolution. Furthermore, while neither prokaryotes nor native yeast do produce cholesterol - on which activity of some GPCRs relies - cholesterol-producing *S. cerevisiae* strains have been engineered. In principle, the disclosed method should be easily transferable to such or any other engineered yeast strains.

### Yeast strain, vectors, cultivation, and expression.

For all experiments *S. cerevisiae* strain BY4741 (*MAT***a** h*is3*Δ*1 leu2*Δ*0 met15*Δ*0 ura3*Δ*0*), obtained from EUROSCARF, was used. Standard expression was performed with pMS03het, derived from p415 GAL1. For more details on different yeast expression vectors see below. BY4741 transformed with pMS03het vectors were cultivated at 30°C in SDD-Leu⁻medium (6.9 g/L yeast nitrogen base without amino acids (Formedium), 690 mg/L complete supplement mixture without leucine (Formedium), 20 g/L glucose, 35 mM sodium citrate tribasic, 35 mM citric acid). For expression, yeast cells in the logarithmic growth phase grown in SDD-Leu⁻ medium at 30°C were centrifuged and subsequently resuspended in SDG-Leu⁻medium (identical to SDD-Leu⁻ but with 20 g/L galactose instead of glucose). Initial OD₆₀₀ was always chosen to be 1.0 after resuspension in SDG-Leu⁻ and expression was performed at 20°C for 24 h.

### Yeast expression vectors

To obtain pMS03het, the α-mating factor prepro sequence was cloned from pPICZα A (Life Technologies) into the multiple cloning site (*Xho*I/*Spe*I) of p415 GAL1. pMS03het contains *Nhe*I/*Bam*HI restriction sites which allow efficient vector linearization for high-efficiency transformation or in-frame cloning of genes preceded by the α-mating factor prepro sequence. For expression of GPCRs with a C-terminal HA tag or fusion to mCherry, vectors pMS03het_HA or pMS03het_mCh were used, respectively. To obtain pMS03het_HA and pMS03het_mCh, sequences coding for HA tag or mCherry were cloned via *Bam*HI into pMS03het.

### Yeast library construction and transformation.

The wild-type gene of rat NTR1 (N-terminally truncated from amino acids 1 - 42) was a kind gift from Reinhard Grisshammer (National Institutes of Health). Wild-type cDNA of human NK1R and human KOR1 was obtained from the Missouri S&T cDNA Resource Center. All wild-type GPCR genes were cloned into pMS03het (*Nhe*I/*Bam*HI)*.* DNA library construction was performed by amplification of wild-type genes or isolated DNA after the first round of evolution with error-prone PCR using the GeneMorph II Random Mutagenesis Kit (Agilent Technologies) according to the manufacturer's protocol. Transformation of BY4741 with DNA libraries was performed by square wave electroporation on a GenePulser Xcell electroporator (Bio-Rad). On average, libraries with a diversity of 5 × 10⁷ - 1 × 10⁸ were obtained. For more details on library construction and high-efficiency transformation see below.

### DNA library construction and high-efficiency transformation

DNA libraries of wild-type GPCRs or isolated versions after the first round of evolution were generated by performing two error-prone PCRs (epPCRs), one epPCR with 20 and one with 25 cycles, of which the obtained products were subsequently pooled. Primers used for epPCR introduced sites homologous to linearized pMS03het (digested with *Nhe*I/*Bam*HI) at each end of the gene.
Forward primer:
   5'-CTAAAGAAGAAGGGGTATCTCTCGAGAAACGTGAGGCGGAAGCGGCTAGC-3';
Reverse primer:
   5'-ATTACATGACTCGACTCGATGCCGACGAGAGCGGCCGCCTATTAGGATCC-3'.

The obtained epPCR products were further amplified by standard PCR with the identical primers in order to obtain enough DNA material for transformation.

High-efficiency transformation by square wave electroporation was performed analogously to a previously published method (Van Deventer & Wittrup (2014), Methods Mol Biol 1131:151-181) with some minor adaptations. Yeast cells were grown in 60 mL YPD at 30°C to an OD₆₀₀ = 1.8 - 2.0. As soon as this cell density was reached, 50 mL of culture were centrifuged, the medium aspirated, and cells were treated in 25 mL conditioning solution (100 mM lithium acetate, 10 mM DTT) at 30°C for 15 min. Subsequently, cells were pelleted, washed in 25 mL cold ddH₂O, pelleted again, and resuspended in cold ddH₂O to a total volume of 500 µL. Henceforward, cells were always kept at 4°C. For one transformation, 250 µL of yeast cells were mixed with 4 µg of linearized pMS03het and 12 µg PCR product and the transformation mixture was transferred to a 2 mm electroporation cuvette. Square wave electroporation was performed with one pulse with a voltage of 500 V and a pulse length of 15 ms. After electroporation, cells were allowed to recover in 5 mL YPD without shaking at 30°C for 1 h. Finally, recovered cells were pelleted, transferred to 500 mL SDD-Leu⁻ for selective growth at 30°C for 20 - 24 h, and stored in glycerol stocks at -80°C. To obtain high-diversity libraries, always two transformations per library were performed.

### Permeabilization of yeast cells and binding of fluorescent ligand.

After expression, cultures were centrifuged, medium was aspirated, and cells were resuspended in TELi buffer (50 mM Tris-HCl pH 9.0 (at 4°C), 1 mM EDTA, 100 mM lithium acetate) at RT. Next, cells were incubated in TELi Buffer supplemented with 50 mM DTT at 20°C for 30 min and subsequently washed twice in cold TELi Buffer. Henceforward, cells were always kept at 4°C. For fluorescent ligand binding, permeabilized cells were incubated with ligand labeled with HiLyte Fluor 488 (AnaSpec) (NTR1 variants: 25 nM fluorescent neurotensin (8-13); NK1R variants: 20 nM fluorescent substance P; KOR1 variants: 10 nM fluorescent dynorphin A (1-11)) in TELi buffer at 4°C without exposure to light for 2 h. After incubation, cells were washed once in TELi buffer prior to measurements. Non-specific binding was determined in the presence of a 1000-fold excess of unlabeled ligand (NTR1 variants: 25 µM neurotensin (8-13) (AnaSpec); NK1R variants: 20 µM substance P (AnaSpec); KOR1 variants: 10 µM dynorphin A (1-11) (GenScript)). For more details on fluorescent ligands see below.

### Fluorescent ligands

All fluorescent ligands were labeled with HiLyte Fluor 488 (Anaspec). Neurotensin (8-13) (KKPYIL) was covalently labeled at the N-terminal amino group. Substance P (RPKPQQFFGLM) was covalently labeled at the amino group of lysine-3. Dynorphin A (1-11) (YGGFLRRIRPK) was covalently labeled at the amino group of lysine-11.

### Flow cytometry and FACS.

Cells fluorescently labeled by ligand binding were kept in TELi buffer for measurements. Flow cytometry was performed on a BD FACSCanto II cytometer (BD Biosciences) or on a BD LSRFortessa cell analyzer (BD Biosciences) and FACS was performed on a BD FACSAria III sorter (BD Biosciences). For analytical measurements always 50,000 events were recorded. In FACS, 3 × 10⁵ - 5 × 10⁵ of the 0.5 - 1.0% most fluorescent cells were sorted into SDD-Leu- medium for subsequent cultivation at 30°C for 24 h. For all samples identical acquisition settings were used in order to allow comparative analysis. Data were analyzed with FlowJo vX.0.7.

### Radioligand binding assays.

RLBAs on whole yeast cells were performed as follows: First, 1 × 10⁸ cells (assuming OD₆₀₀ = 1.0 corresponds to 10⁷ cells/mL) were harvested after expression and treated by consecutive washing first in ddH₂O, then SPH1 buffer (1 M sorbitol, 25 mM EDTA, 50 mM DTT, pH 8.0), and finally in 1 M sorbitol. Next, cells were resuspended in SPH2 buffer (1 M sorbitol, 1 mM EDTA, 10 mM potassium citrate tribasic, pH 5.8) and cell wall digestion was performed by addition of 6 U/mL Zymolyase 20T (AMS Biotechnology) followed by incubation at 30°C for 30 min. Henceforward, cells were kept at 4°C. Subsequently, cells were incubated at 4°C for 2 h in 50 mM Tris-HCl pH 7.4 (at 4°C) containing [³H]-labeled ligand (NTR1 variants: 20 nM [3,11-Tyrosyl-3,5-³H(N)]-neurotensin (Perkin Elmer); NK1R variants: 15 nM [Leucyl-3,4,5-³H(N)]-substance P (Perkin Elmer); KOR1 variants: 15 nM [15,16-³H]-diprenorphine (Perkin Elmer)). Nonspecific binding was determined in the presence of a 1000-fold excess of unlabeled ligand (NTR1 variants: 20 µM neurotensin (8-13) (AnaSpec); NK1R variants: 15 µM substance P (AnaSpec); KOR1 variants: 15 µM diprenorphine (Tocris Bioscience)). After incubation, cells were filtered on MultiScreen filter plates (Merck Millipore) with a vacuum manifold, filters were washed four times with cold 50 mM Tris-HCl pH 7.4 (at 4°C), transferred to Isoplate-96 scintillation plates (Perkin Elmer), dried at 65°C for 2 h, and Optiphase Supermix scintillation cocktail (Perkin Elmer) was added. RLBA measurements were performed on a 1450 MicroBeta Plus liquid scintillation counter (Wallac).

RLBAs on whole *Sf*9 cells were performed as described previously (Schlinkmann et al. (2012), J Mol Biol 422:414-428; Egloff et al. (2014), Proc Natl Acad Sci USA 111:E655-62.). Whole cells were incubated in binding buffer (50 mM Tris-HCl pH 7.4 (at 4°C), 1 mM EDTA, 0.1% (w/v) BSA and 40 µg/mL bacitracin) containing 15 nM [³H]-labeled ligand. Nonspecific binding was determined in the presence of 15 µM unlabeled ligand. The same ligands as for RLBAs with yeast cells were used.

### Quantitative Western blot.

After expression, 4 × 10⁷ cells (assuming OD₆₀₀ = 1.0 corresponds to 10⁷ cells/mL) for each sample were centrifuged and whole cell protein extraction was performed according to a previously published protocol (Zhang et al. (2011), Yeast 28:795-798.). Protein detection was performed with the primary antibodies rabbit anti-HA (Sigma-Aldrich, H6908) and mouse anti-actin (Abcam, ab8224) and the secondary antibodies goat anti-rabbit conjugated to Alexa Fluor 680 (Life Technologies, A-21076) and donkey anti-mouse conjugated to IRDye800 (Rockland Immunochemicals, 610-732-124). Image acquisition was performed on an Odyssey system (LI-COR Biosciences) and quantification was performed with Image Studio Lite version 3.1.4 (LI-COR Biosciences). For more details see below.

### Yeast whole cell protein extraction, SDS-PAGE, and Western blot

For whole cell protein extraction, samples after expression were centrifuged, medium was aspirated, and pelleted cells were resuspended in 500 µL 2 M lithium acetate for incubation on ice for 5 min. Next, cells were pelleted, the supernatant was removed, 100 µL of 0.4 M NaOH were added, and samples were incubated on ice for 5 min. After incubation, the samples were centrifuged, the supernatant was removed and the cell pellets were resuspended in 200 µL reducing NuPAGE LDS sample buffer (Life Technologies). Samples were incubated at 20°C for 15 min, centrifuged, and 5 µL of each sample were run on a NuPAGE Novex 4-12% Bis-Tris protein gel (Life Technologies) in NuPAGE MES SDS running buffer (Life Technologies). Wet blotting was performed onto Immobilon-FL membranes (Merck Millipore). Blocking of membranes was performed in 1× Casein blocking buffer (Sigma-Aldrich) in PBS at RT for 20 min. Antibody binding was performed in 1× Casein blocking buffer in PBST (PBS, 0.05% (v/v) Tween-20) at RT for 1 h, and PBST was used for all membrane washing steps. Primary rabbit anti-HA antibody was used at a dilution of 1:5,000, primary mouse anti-actin antibody at a dilution of 1:1,000, and the secondary antibodies (goat anti-rabbit conjugated to Alexa Fluor 680 and donkey anti-mouse conjugated to IRDye800) both at a dilution of 1:10,000.

### Confocal fluorescence microscopy.

Yeast cells were permeabilized and binding of fluorescent neurotensin was performed as described above. After washing, cells were transferred into Nunc Lab-Tek II chambered coverglasses (Thermo Scientific) and confocal microscopy was performed on a Leica TCS SP5 microscope (Leica Microsystems). For all samples magnification was 630-fold and identical acquisition settings were used in order to allow comparative analysis.

### Spodoptera frugiperda (Sf9) vectors and expression.

Wild-type and evolved receptor constructs were amplified by PCR from yeast expression vector pMS03het and cloned via SLIC into a modified MultiBac pFL vector. The vector designated as pFL_mFLAG_His₁₀_TEV_SLIC contains an expression cassette with an N-terminal melittin signal sequence followed by a FLAG tag, a deca-histidine tag, a TEV protease cleavage site, and a SLIC cloning site. *E. coli* DH10 EMBacY cells were transformed with pFL vectors containing the different receptor genes and the resulting baculovirus genome was isolated. For details on generation of recombinant baculovirus and baculovirus-infected insect cell stocks (BIICs) see below. Expression was performed in Sf-900 II SFM medium (Life Technologies) by infection of *Sf*9 cells at a density of 3 × 10⁶ cells/mL with 100-fold diluted BIIC stocks and cultivation at 27°C for 4 d. After expression, cells were harvested by centrifugation, washed in cold PBS and stored at -80°C until use.

### Generation of recombinant baculovirus and baculovirus-infected insect cell stocks (BIICs).

Recombinant baculovirus was generated by transfecting 8 × 10⁵ *Sf*9 cells in 2 mL of Sf-900 II SFM medium (Life Technologies) using 8 µL Cellfectin II reagent (Life Technologies). After 4 h of incubation in a humidified incubator at 27°C, the transfection medium was removed and replaced by 2 mL of fresh Sf-900 II SFM. V0 viral stock was harvested after 5 d at 27°C and used to generate V1 high-titer virus stock (10⁸ - 10⁹ viral particles per mL). V1 virus stock was then used to generate BIICs. Briefly, *Sf*9 cells at a density of 10⁶ cells/mL were infected with a multiplicity of infection (MOI) of 5, incubated for 24 h in suspension, harvested and frozen at -80°C in aliquots in Sf-900 II SFM containing Penicillin-Streptomycin (Life Technologies) and 10% (v/v) DMSO.

### [³⁵S]-GTPγS binding assay.

Membranes used for [³⁵S]-GTPγS binding assays were isolated as previously described (Egloff et al. (2014), Proc Natl Acad Sci USA 111:E655-62). Briefly, cells were disrupted by shear force after osmotic shock. In order to reduce unspecific [³⁵S]-GTPγS binding, membranes were washed in a urea containing buffer. The isolated membranes were frozen in aliquots and stored at -80°C. Receptor levels on urea-washed membranes were determined by radioligand binding assays as described above.

The [³⁵S]-GTPγS binding assay was performed as previously described (Egloff et al. (2014), Proc Natl Acad Sci USA 111:E655-62). Briefly, 1 nM GPCR in urea-washed membranes and 100 nM G protein (Gαi₁β₁γ₁, purified according to (Rasmussen et al. (2011), Nature 477:549-555.)) in assay buffer (50 mM Tris-HCl pH 7.4 (at 4°C), 1 mM EDTA, 100 mM NaCl, 1 mM DTT, 3 mM MgSO₄, 0.3% (w/v) BSA, 2 µM GDP, 4 nM [³⁵S]-GTPγS (Perkin Elmer)) were incubated at 25°C for 20 min in the presence or absence of 200 µM substance P (AnaSpec). Background counts arising from buffer, GPCR and G protein alone have been taken into account and subtracted. Therefore, given counts represent the GPCR-induced [³⁵S]-GTPγS binding to G protein in the presence and absence of agonist.

### Purification of NK1R variant NK1R-Y09

All steps were performed at 4°C. Frozen Sf9 cells were thawed and swelled in a hypotonic buffer (10 mM HEPES pH 7.4, 20 mM KCI, 10 mM MgCl₂, complete ULTRA EDTA-free tablets (Roche), 1 µM substance P) for 1 h. Cell membranes were then disrupted by homogenization (Dounce homogenizer) and collected by centrifugation at 130,000 rcf. The isolated membranes were extensively washed twice by repeated homogenization (Dounce homogenizer) and centrifugation in hypotonic buffer, followed by three repetitions of this procedure using a hypertonic buffer (10 mM HEPES pH 7.4, 1 M NaCl, 20 mM KCI, 10 mM MgCl₂, complete ULTRA EDTA-free tablets, 1 µM substance P). Purified membranes were resuspended in solubilization buffer (30 mM HEPES pH 7.4, 150 mM NaCl, 10 mM MgCl₂, complete ULTRA EDTA-free tablets, 1 µM substance P, 2 mg/mL iodoacetamide (Sigma)) and stirred for 45 min. Membranes were then solubilized in 1.5% (w/v) n-dodecyl-β-D-maltopyranoside (DDM, Anatrace) and 0.3% (w/v) cholesteryl hemisuccinate (CHS, Sigma). After 3 h of stirring, non-solubilized material was removed by centrifugation (130,000 rcf, 40 min, 4°C). The supernatant was adjusted to contain an end concentration of 800 mM NaCl and 25 mM imidazole before being incubated overnight with 1.5 mL TALON Superflow resin (Clontech). The protein-bound resin was transferred into gravity flow columns and then washed with 10 column volumes (CV) each of Wash 1 buffer (25 mM HEPES pH 7.4, 800 mM NaCl, 10 mM MgCl₂, 25 mM imidazole, 10% (v/v) glycerol, 0.5 µM substance P, 0.3%/0.06% DDM/CHS), Wash 2 buffer (25 mM HEPES pH 7.4, 400 mM NaCl, 10 mM MgCl₂, 40 mM imidazole, 10% (v/v) glycerol, 0.5 µM substance P, 0.2%/0.04% DDM/CHS, 10 mM ATP), and Wash 3 buffer (25 mM HEPES pH 7.4, 200 mM NaCl, 40 mM imidazole, 10% (v/v) glycerol, 0.5 µM substance P, 0.2%/0.04% DDM/CHS). NK1R-Y09 was eluted in 4 CV of elution buffer (25 mM HEPES pH 7.4, 200 mM NaCl, 300 mM imidazole, 10% (v/v) glycerol, 0.5 µM substance P, 0.2%/0.04% DDM/CHS). Purified receptor was concentrated to 0.5 mL with 100 kDa molecular weight cut-off Vivaspin centrifuge concentrators (Sartorius Stedim Biotech). Size-exclusion chromatography (SEC) was performed on an Aekta Pure FPLC system (GE Healthcare) with a Superdex S200 Increase 10/300 GL column (GE Healthcare) equilibrated with SEC buffer (20 mM HEPES pH 7.4, 150 mM NaCl, 0.05%0.01% DDM/CHS, 0.1 µM substance P).

### Example 2: Directed evolution of GPCRs in yeast

Directed evolution of GPCRs towards higher expression levels in *Escherichia coli* has been established previously (Sarkar et al. (2008), Proc Natl Acad Sci USA 105:14808-14813; Dodevski & Plückthun (2011), J Mol Biol 408:599-615). This invention discloses the analogous method for directed evolution in the yeast *Saccharomyces cerevisiae.* Several GPCRs have been evolved with this method in *S. cerevisiae,* leading to high functional expression in yeast as well as in insect cells and outperforming expression levels obtained from variants evolved in the *E. coli*-based system. Usually, only two rounds of evolution were required to obtain highly expressed variants.

Interestingly, the yeast strain used can also be adapted towards increased GPCR production. This is automatically induced during evolution by repetitive sorting with fluorescence-activated cell sorting (FACS) and gradually arises in the course of selection. Thus, obtained expression levels of selected libraries are a result of GPCR evolution combined with host adaption. If individual GPCR variants are expressed in newly transformed yeast cells, the average expression level will be lower than for selected libraries, since these cells were not adapted. However, simply by repetitive FACS selections the adaption can be induced for any evolved variant.

### GPCR Expression Constructs

Several different GPCR expression constructs are available. All vectors are derived from p415 GAL1. The vectors are maintained as low-copy plasmids in *S. cerevisiae* providing a functional LEU2 gene for selective growth of strains auxotrophic for leucine and can be easily re-isolated from yeast after transformation. Being shuttle-vectors, they can be propagated as high-copy vectors in *E. coli,* in which correspondingly all cloning steps are performed.

Every vector encodes the α-mating factor prepro sequence preceding the cloning site for insertion of the GPCR gene. Cloning is done via *Nhe*I and *Bam*HI restriction sites, allowing in-frame insertion of the gene of interest. Expression is under control of the inducible GAL1 promoter. The different vectors vary in the sequences after the cloning site. The standard vector used for evolution, pMS03het, does not encode any tag or protein after the cloning site, thus GPCRs without any fusions are produced with pMS03het. The other vectors encode constructs containing a C-terminal cleavable deca-histidine-tag (pMS03het_3CHis10), a HA-tag (pMS03het HA), a fusion to mCherry (pMS03het_mCh), or an AviTag^{™} (pMS03het_Avi).

### Permeabilization of Yeast Cells for Ligand Binding

This section describes the permeabilization of yeast cells for ligand binding experiments. Conditioning with TELi buffer and DTT permeabilizes the cell wall and allows diffusion of ligands to the GPCRs in the cell membrane. The advantage of this permeabilization method is that the cells remain viable and are not as fragile as spheroplasts. Therefore, this procedure can be used for analytical flow cytometry and FACS, as well as for any other application in which ligand binding is detected (e.g. confocal fluorescence microscopy, RLBA).

### Protocol:

- Cells are collected after expression by centrifugation (5,000 rcf, 10 min, RT) and the supernatant is aspirated.
- Cells are resuspended in 1 mL TELi buffer at RT, centrifuged (4,000 rcf, 3 min, RT) and the supernatant is aspirated.
- Cells are resuspended in 900 µL TELi buffer at RT and 100 µL freshly prepared and filter-sterilized 0.5 M DTT (dissolved in 100 mM lithium acetate solution) is added. This gives a final concentration of 50 mM DTT.
- Cells are incubated at 20°C in a thermomixer, shaking with 700 rpm for 30 min.
- Cells are centrifuged (4,000 rcf, 3 min, 4°C) and the supernatant is aspirated.
- Cells must from now on always be kept on ice.
- Cells are resuspended in 1 mL cold TELi buffer, centrifuged (4,000 rcf, 3 min, 4°C) and the supernatant is aspirated.
- Cells are resuspended in cold TELi buffer (the volume is chosen according to the experimental needs).

### Measurement of GPCR Expression with Flow Cytometry

Detection of functional GPCR expression at the cell surface can in principle be performed by fluorescent ligand binding measured in flow cytometry. Upon incubation with fluorescent ligand, only functionally expressed GPCRs located in the plasma membrane will bind the ligand. After removal of unbound ligand by washing, ligand binding is detected by measuring fluorescence intensity. However, the yeast cell wall acts as a protective barrier, not allowing larger molecules like peptide ligands to pass through. Therefore, yeast cells need to be permeabilized prior to incubation with fluorescent ligand. Next to analytical quantification of functional expression levels, fluorescent ligand binding is also used for selections with FACS. Cells expressing a variant with a high-expression phenotype will correspondingly exhibit a higher fluorescence intensity. By sorting the most fluorescent cells, highly expressing GPCR variants can be isolated.

While incubation with fluorescent ligand allows measurement of the total signal, it is important to determine the non-specific signal as well. This can be done by incubation of fluorescent ligand with an excess of non-labeled ligand in a competition binding experiment. The obtained non-specific signal in such measurements depicts the background. Usually, GPCR expression in yeast leads to two subpopulation of cells. Whereas one fraction of cells shows surface expression of active GPCRs, there is also a subpopulation for which no active surface expression is observed. Typically, this fraction of non-expressing cells decreases in adapted strains.

### Workflow of Selections with FACS

An overview of the workflow of selections is depicted in Fig. 1. For directed evolution of GPCRs the selection starts with the generation of a library. A new DNA library is created by random mutagenesis with error-prone PCR (epPCR) on a GPCR gene. The DNA library has to be amplified by PCR (ampPCR) with special primers generating an insert suitable for high-efficiency transformation. Once a yeast library has been created by transformation with the desired efficiency, selections with FACS can be performed. Typically, 5 rounds of FACS are performed. Once the selections with FACS are done, the DNA of the selected clones is isolated. At this step, the selected clones can be analyzed. If desired, another round of directed evolution can be performed by creating a new library from the selected clones by epPCR and subsequent selections with FACS. In previous directed evolutions of GPCRs, two rounds of evolution - meaning two randomizations, each followed by five selections with FACS - were required to obtain highly increased expression levels.

For adaptation of strains expressing an evolved GPCR variant, the workflow is almost identical. The only difference is that no library needs to be prepared. Strain adaptation starts with a single clone expressing the GPCR variant of choice which is subjected to 5 rounds of selection with FACS. After the selections, glycerol stocks of the adapted strains can be prepared.

## Claims

1. A method for increasing expression of functional G protein-coupled receptors (GPCRs) in eukaryotic cells, wherein said functional GPCRs are capable of ligand binding, and wherein the method comprises the steps of:
a) providing a plurality of yeast cells, wherein each of said yeast cells comprises a nucleic acid sequence member of a randomized mutant library, and said nucleic acid sequence member is expressed as a GPCR in the plasma membrane in said plurality of yeast cells,
b) permeabilizing the cell wall of said plurality of yeast cells in a permeabilization step, wherein the permeabilization step comprises exposing the plurality of yeast cells to a chemical treatment, yielding a plurality of viable permeabilized cells, wherein the chemical treatment is a buffer of alkaline pH comprising lithium ions, a reducing agent and/or a chelating agent,
c) contacting said plurality of viable permeabilized cells in a labelling step with a ligand capable of binding to said GPCR, wherein saidligand comprises a detectable label, yielding a plurality of viable labelled cells,
d) washing said plurality of viable labelled cells in a washing step,
e) selecting a subset of said plurality of viable labelled cells as a function of detectable label present in said plurality of viable labelled cells in a selection step, yielding a selection of viable cells, wherein said detectable label is a fluorescent dye and said selection step is accomplished by fluorescent cell sorting,
f) after said selection step e) said selection of viable cells is expanded in an expansion step, yielding an expanded selection of viable cells and said expanded selection of viable cells is subjected to said steps b) to e) in this sequential order,
g) repeating step f) at least 1, 2, 3, 4, 5, 6 or 7 times, and
h) isolating an expressed nucleic acid sequence from the expanded selection of viable cells of step f) in an isolation step, wherein the expanded selection of viable cells of step g) has increased expression of functional GPCRs as compared to the plurality of yeast cells of step a).

2. The method according to claim 1, wherein the expanded selection of viable cells of step g) have increased surface expression of functional GPCRs as compared to the plurality of yeast cells of step a).

3. The method according to claim 1 or 2, wherein the expanded selection of viable cells of step g) have an ability for expression of at least 100,000 total functional GPCRs per cell.

4. The method according to any one of the preceding claims, wherein the expanded selection of viable cells of step g) have a decreased subpopulation of non-expressing GPCR yeast cells as compared to the plurality of yeast cells of step a).

5. The method according to any one of the preceding claims, wherein the expanded selection of viable cells of step g) have a decreased subpopulation of cells expressing intracellularly retained receptor as compared to the plurality of yeast cells of step a).

6. The method according to any one of the preceding claims, wherein the expanded selection of viable cells of step g) have a greater amount of surface expression of functional GPCRs per total amount of receptor produced relative to the amount of surface expression of functional GPCRs per total amount of receptor produced of the plurality of yeast cells of step a).

7. The method according to claim 1, wherein:
i. the expressed nucleic acid obtained in said isolation step h) is introduced and expressed in a plurality of yeast cells,
ii. said plurality of yeast cells is subjected to said steps b) to h) according to claim 1, and
iii. steps i. and ii. are performed at least 1, 2, 3, 4, 5, 6 or 7 times.

8. The method according to any one of the preceding claims, wherein said expressed nucleic acid obtained in said isolation step h) is amplified by a process introducing mutations into the amplified sequence, yielding a second library of nucleic acid sequences and:
i. said second library of nucleic acid sequences is transferred to said plurality of yeast cells, and
ii. said plurality of yeast **c**ells is submitted to the method according to claim 1 or claim 7.

9. A method for the selection of an adapted yeast cell with the ability for high expression levels of functional GPCRs, comprising the steps of:
a. providing a plurality of yeast cells, wherein each of said yeast cells comprises a nucleic acid sequence member of a randomized mutant library of expressed nucleic acid sequences, and said nucleic acid sequence member is expressed as a GPCR in the plasma membrane in said plurality of yeast cells,
b. permeabilizing the cell wall of said plurality of yeast cells in a permeabilization step, wherein the permeabilization step comprises exposing the plurality of yeast cells to a chemical treatment, yielding a plurality of viable permeabilized cells, wherein the chemical treatment is a buffer of alkaline pH comprising lithium ions, a reducing agent and/or a chelating agent,
c. contacting said plurality of viable permeabilized cells in a labelling step with a ligand capable of binding to said GPCR, wherein saidligand comprises a detectable label, yielding a plurality of viable labelled cells,
d. washing said plurality of viable labelled cells in a washing step,
e. selecting a subset of said plurality of viable labelled cells as a function of detectable label present in said plurality of viable labelled cells in a selection step, yielding a selection of viable cells, wherein said detectable label is a fluorescent dye and said selection step is accomplished by fluorescent cell sorting,
f. expanding said selection of viable cells in an expansion step, yielding an expanded selection of viable cells,
g. submitting said expanded selection of viable cells to steps b. to f. at least 1, 2, 3, 4, 5, 6 or 7times,
h. submitting said expanded selection of viable cells to steps b. to e., and
i. selecting a subset of said expanded selection of viable cells as a function of detectable label present in said plurality of viable labelled cells, yielding said adapted yeast cell with the ability for high expression levels of functional GPCRs from said expanded selection of cells.

## Patentansprüche

1. Verfahren zur Steigerung der Expression funktioneller G-Protein-gekoppelter Rezeptoren (GPCRs) in eukaryotischen Zellen, wobei die funktionellen GPCRs zur Ligandenbindung fähig sind und wobei das Verfahren die folgenden Schritte umfasst:
a) Bereitstellen einer Vielzahl von Hefezellen, wobei jede der Hefezellen ein Nukleinsäuresequenzmitglied einer randomisierten Mutantenbibliothek umfasst und das Nukleinsäuresequenzmitglied als ein GPCR in der Plasmamembran in der Vielzahl von Hefezellen exprimiert wird,
b) Permeabilisieren der Zellwand der Vielzahl von Hefezellen in einem Permeabilisierungsschritt, wobei der Permeabilisierungsschritt das Aussetzen der Vielzahl von Hefezellen gegenüber einer chemischen Behandlung umfasst, wodurch eine Vielzahl lebensfähiger permeabilisierter Zellen entsteht, wobei die chemische Behandlung ein Puffer mit alkalischem pH-Wert ist, der Lithiumionen, ein Reduktionsmittel und/oder einen Chelatbildner umfasst,
c) Kontaktieren der Vielzahl lebensfähiger permeabilisierter Zellen in einem Markierungsschritt mit einem Liganden, der an den GPCR binden kann, wobei der Ligand eine nachweisbare Markierung umfasst, wodurch eine Vielzahl lebensfähiger markierter Zellen entsteht,
d) Waschen der Vielzahl lebensfähiger markierter Zellen in einem Waschschritt,
e) Auswählen einer Untergruppe der Vielzahl lebensfähiger markierter Zellen als Funktion der in der Vielzahl lebensfähiger markierter Zellen vorhandenen nachweisbaren Markierung in einem Auswahlschritt, wodurch eine Auswahl lebensfähiger Zellen entsteht, wobei die nachweisbare Markierung ein Fluoreszenzfarbstoff ist und der Selektionsschritt durch fluoreszierende Zellsortierung durchgeführt wird,
f) nach dem Auswahlschritt e) wird die Auswahl lebensfähiger Zellen in einem Expansionsschritt erweitert, wodurch eine erweiterte Auswahl lebensfähiger Zellen entsteht, und die erweiterte Auswahl lebensfähiger Zellen wird den Schritten b) bis e) in dieser Reihenfolge unterzogen,
g) Wiederholen des Schritts f) mindestens 1, 2, 3, 4, 5, 6 oder 7 Mal, und
h) Isolieren einer exprimierten Nukleinsäuresequenz aus der erweiterten Auswahl lebensfähiger Zellen aus Schritt f) in einem Isolierungsschritt,
wobei die erweiterte Auswahl lebensfähiger Zellen aus Schritt g) eine erhöhte Expression von funktionellen GPCRs im Vergleich zu der Vielzahl von Hefezellen aus Schritt a) aufweist.

2. Verfahren nach Anspruch 1, wobei die erweiterte Auswahl lebensfähiger Zellen aus Schritt g) im Vergleich zu der Vielzahl von Hefezellen aus Schritt a) eine erhöhte Oberflächenexpression funktioneller GPCRs aufweist.

3. Verfahren nach Anspruch 1 oder 2, wobei die erweiterte Auswahl lebensfähiger Zellen aus Schritt g) die Fähigkeit zur Expression von mindestens 100.000 insgesamt funktionellen GPCRs pro Zelle aufweist.

4. Verfahren nach einem der vorstehenden Ansprüche, wobei die erweiterte Auswahl lebensfähiger Zellen aus Schritt g) im Vergleich zu der Vielzahl von Hefezellen aus Schritt a) eine verringerte Subpopulation von nicht exprimierenden GPCR-Hefezellen aufweist.

5. Verfahren nach einem der vorstehenden Ansprüche, wobei die erweiterte Auswahl lebensfähiger Zellen aus Schritt g) im Vergleich zu der Vielzahl von Hefezellen aus Schritt a) eine verringerte Subpopulation von Zellen aufweist, die intrazellulär zurückgehaltene Rezeptoren exprimieren.

6. Verfahren nach einem der vorstehenden Ansprüche, wobei die erweiterte Auswahl lebensfähiger Zellen aus Schritt g) eine größere Menge an Oberflächenexpression funktioneller GPCRs pro Gesamtmenge des produzierten Rezeptors im Verhältnis zu der Menge an Oberflächenexpression funktioneller GPCRs pro Gesamtmenge des produzierten Rezeptors der Vielzahl von Hefezellen aus Schritt a) aufweist.

7. Verfahren nach Anspruch 1, wobei:
i. die in dem Isolierungsschritt h) erhaltene exprimierte Nukleinsäure in eine Vielzahl von Hefezellen eingeführt und darin exprimiert wird;
ii. die Vielzahl von Hefezellen den genannten Schritten b) bis h) nach Anspruch 1 unterzogen wird, und
ii. die Schritte i. und ii. mindestens 1, 2, 3, 4, 5, 6 oder 7 Mal durchgeführt werden.

8. Verfahren nach einem der vorstehenden Ansprüche, wobei die in dem Isolierungsschritt h) erhaltene exprimierte Nukleinsäure durch ein Verfahren amplifiziert wird, bei dem Mutationen in die amplifizierte Sequenz eingeführt werden, wodurch eine zweite Bibliothek von Nukleinsäuresequenzen entsteht, und:
i. die zweite Bibliothek von Nukleinsäuresequenzen wird auf die Vielzahl von Hefezellen übertragen, und
ii. die Vielzahl von Hefezellen dem Verfahren nach Anspruch 1 oder Anspruch 7 unterzogen wird.

9. Verfahren zur Selektion einer angepassten Hefezelle mit der Fähigkeit zu hohen Expressionsniveaus funktioneller GPCRs, umfassend die folgenden Schritte:
a. Bereitstellen einer Vielzahl von Hefezellen, wobei jede der Hefezellen ein Nukleinsäuresequenzmitglied einer randomisierten Mutantenbibliothek exprimierter Nukleinsäuresequenzen umfasst und das Nukleinsäuresequenzmitglied als GPCR in der Plasmamembran in der Vielzahl von Hefezellen exprimiert wird,
b. Permeabilisieren der Zellwand der Vielzahl von Hefezellen in einem Permeabilisierungsschritt, wobei der Permeabilisierungsschritt das Aussetzen der Vielzahl von Hefezellen gegenüber einer chemischen Behandlung umfasst, wodurch eine Vielzahl lebensfähiger permeabilisierter Zellen entsteht, wobei die chemische Behandlung ein Puffer mit alkalischem pH-Wert ist, der Lithiumionen, ein Reduktionsmittel und/oder einen Chelatbildner umfasst,
c. Kontaktieren der Vielzahl lebensfähiger permeabilisierter Zellen in einem Markierungsschritt mit einem Liganden, der an den GPCR binden kann, wobei der Ligand eine nachweisbare Markierung umfasst, wodurch eine Vielzahl lebensfähiger markierter Zellen entsteht,
d. Waschen der Vielzahl lebensfähiger markierter Zellen in einem Waschschritt,
e. Auswählen einer Untergruppe der Vielzahl lebensfähiger markierter Zellen als Funktion der in der Vielzahl lebensfähiger markierter Zellen vorhandenen nachweisbaren Markierung in einem Auswahlschritt, wodurch eine Auswahl lebensfähiger Zellen entsteht, wobei die nachweisbare Markierung ein Fluoreszenzfarbstoff ist und der Selektionsschritt durch fluoreszierende Zellsortierung durchgeführt wird,
f. Erweitern der Auswahl lebensfähiger Zellen in einem Expansionsschritt, wodurch eine erweiterte Auswahl lebensfähiger Zellen entsteht,
g. Unterziehen der erweiterten Auswahl lebensfähiger Zellen den Schritten b. bis f. mindestens 1, 2, 3, 4, 5, 6 oder 7 Mal,
h. Unterziehen der erweiterten Auswahl lebensfähiger Zellen den Schritten b. bis e., und
i. Auswählen einer Untergruppe der erweiterten Auswahl lebensfähiger Zellen als Funktion der nachweisbaren Markierung, die in der Vielzahl lebensfähiger markierter Zellen vorhanden ist, wodurch die angepasste Hefezelle mit der Fähigkeit zu hohen Expressionsniveaus funktioneller GPCRs aus der erweiterten Auswahl von Zellen entsteht.

## Revendications

1. Procédé destiné à augmenter l'expression de récepteurs couplés aux protéines G (GPCR) fonctionnels dans des cellules eucaryotes, dans lequel lesdits GPCR fonctionnels sont aptes à la fixation du ligand, et dans lequel le procédé comprend les étapes de :
a) fournir une pluralité de cellules de levure, dans laquelle chacune desdites cellules de levure comprend une séquence d'acide nucléique membre d'une banque de mutants aléatoires, et ledit membre de séquence d'acide nucléique est exprimé en tant que GPCR dans la membrane plasmique dans ladite pluralité de cellules de levure,
b) perméabiliser la paroi cellulaire de ladite pluralité de cellules de levure dans une étape de perméabilisation, l'étape de perméabilisation comprenant l'exposition de la pluralité de cellules de levure à un traitement chimique, donnant une pluralité de cellules viables perméabilisées, le traitement chimique étant un tampon de pH alcalin comprenant des ions lithium, un agent réducteur et/ou un agent chélatant,
c) mettre en contact ladite pluralité de cellules viables perméabilisées dans une étape de marquage avec un ligand apte à se fixer audit GPCR, dans lequel ledit ligand comprend un marqueur détectable, donnant une pluralité de cellules viables marquées,
d) laver ladite pluralité de cellules viables marquées dans une étape de lavage,
e) sélectionner un sous-ensemble de ladite pluralité de cellules viables marquées en fonction du marqueur détectable présent dans ladite pluralité de cellules viables marquées dans une étape de sélection, donnant une sélection de cellules viables, dans lequel ledit marqueur détectable est un colorant fluorescent et ladite étape de sélection est accomplie par tri cellulaire par fluorescence,
f) après ladite étape de sélection e) ladite sélection de cellules viables est soumise à une expansion dans une étape d'expansion, donnant une sélection de cellules viables ayant subi une expansion et ladite sélection de cellules viables ayant subi une expansion est soumise auxdites étapes b) à e) dans cet ordre séquentiel,
g) répéter l'étape f) au moins 1, 2, 3, 4, 5, 6 ou 7 fois, et
h) isoler une séquence d'acide nucléique exprimée dans la sélection de cellules viables ayant subi une expansion de l'étape f) dans une étape d'isolement, dans lequel la sélection de cellules viables ayant subi une expansion de l'étape g) présente une expression accrue de GPCR fonctionnels par comparaison à la pluralité de cellules de levure de l'étape a).

2. Procédé selon la revendication 1, dans lequel la sélection de cellules viables ayant subi une expansion de l'étape g) présente une expression de surface accrue de GPCR fonctionnels par comparaison à la pluralité de cellules de levure de l'étape a).

3. Procédé selon la revendication 1 ou la revendication 2, dans lequel la sélection de cellules viables ayant subi une expansion de l'étape g) présente une aptitude à l'expression d'au moins 100 000 GPCR fonctionnels totaux par cellule.

4. Procédé selon l'une quelconque des revendications précédentes, dans lequel la sélection de cellules viables ayant subi une expansion de l'étape g) présente une sous-population diminuée de cellules de levure n'exprimant pas de GPCR par comparaison à la pluralité de cellules de levure de l'étape a).

5. Procédé selon l'une quelconque des revendications précédentes, dans lequel la sélection de cellules viables ayant subi une expansion de l'étape g) présente une sous-population diminuée de cellules exprimant un récepteur à rétention intracellulaire par comparaison à la pluralité de cellules de levure de l'étape a).

6. Procédé selon l'une quelconque des revendications précédentes, dans lequel la sélection de cellules viables ayant subi une expansion de l'étape g) présente une quantité plus grande d'expression de surface de GPCR fonctionnels par quantité totale de récepteur produit par rapport à la quantité d'expression de surface de GPCR fonctionnels par quantité totale de récepteur produit de la pluralité de cellules de levure de l'étape a).

7. Procédé selon la revendication 1, dans lequel :
i. l'acide nucléique exprimé obtenu dans ladite étape d'isolement h) est introduit et exprimé dans une pluralité de cellules de levure,
ii. ladite pluralité de cellules de levure est soumise auxdites étapes b) à h) selon la revendication 1, et
iii. les étapes i. et ii. sont réalisées au moins 1, 2, 3, 4, 5, 6 ou 7 fois.

8. Procédé selon l'une quelconque des revendications précédentes, dans lequel ledit acide nucléique exprimé obtenu dans ladite étape d'isolement h) est amplifié par un processus introduisant des mutations dans la séquence amplifiée, donnant une seconde banque de séquences d'acides nucléiques et :
i. ladite seconde banque de séquences d'acides nucléiques est transférée à ladite pluralité de cellules de levure, et
ii. ladite pluralité de cellules de levure est soumise au procédé selon la revendication 1 ou la revendication 7.

9. Procédé pour la sélection d'une cellule de levure adaptée avec l'aptitude à des niveaux élevés d'expression de GPCR fonctionnels, comprenant les étapes de :
a. fournir une pluralité de cellules de levure, dans laquelle chacune desdites cellules de levure comprend une séquence d'acide nucléique membre d'une banque de mutants aléatoires de séquences d'acide nucléique exprimées, et ledit membre de séquence d'acide nucléique est exprimé en tant que GPCR dans la membrane plasmique dans ladite pluralité de cellules de levure,
b. perméabiliser la paroi cellulaire de ladite pluralité de cellules de levure dans une étape de perméabilisation, dans laquelle l'étape de perméabilisation comprend l'exposition de la pluralité de cellules de levure à un traitement chimique, donnant une pluralité de cellules viables perméabilisées, dans laquelle le traitement chimique est un tampon de pH alcalin comprenant des ions lithium, un agent réducteur et/ou un agent chélatant,
c. mettre en contact ladite pluralité de cellules viables perméabilisées dans une étape de marquage avec un ligand apte à se fixer audit GPCR, dans lequel ledit ligand comprend un marqueur détectable, donnant une pluralité de cellules viables marquées,
d. laver ladite pluralité de cellules viables marquées dans une étape de lavage,
e. sélectionner un sous-ensemble de ladite pluralité de cellules viables marquées en fonction du marqueur détectable présent dans ladite pluralité de cellules viables marquées dans une étape de sélection, donnant une sélection de cellules viables, dans lequel ledit marqueur détectable est un colorant fluorescent et ladite étape de sélection est accomplie par tri cellulaire par fluorescence,
f. soumettre ladite sélection de cellules viables à une expansion dans une étape d'expansion, donnant une sélection de cellules viables ayant subi une expansion,
g. soumettre ladite sélection de cellules viables ayant subi une expansion aux étapes b. à f., au moins 1, 2, 3, 4, 5, 6 ou 7 fois,
h. soumettre ladite sélection de cellules viables ayant subi une expansion aux étapes b. à e., et
i. sélectionner un sous-ensemble de ladite sélection de cellules viables ayant subi une expansion en fonction du marqueur détectable présent dans ladite pluralité de cellules viables marquées, donnant ladite cellule de levure adaptée avec l'aptitude à des niveaux élevés d'expression de GPCR fonctionnels à partir de ladite sélection de cellules ayant subi une expansion.
